# EUROPEAN PATENT SPECIFICATION

(11) **EP 1 181 269 B1**
(45) Date of publication and mention of the grant of the patent: **25.02.2004**
(21) Application number: 00936388.8
(22) Date of filing: 26.05.2000
(51) Int. Cl.: C07C 309/73, C07C 309/75, C07C 309/76, C07C 311/21, C07C 311/29

(54) **CELL PROLIFERATION INHIBITORS**
ZELLPROLIFERATION INHIBITOREN
INHIBITEURS DE PROLIFERATION CELLULAIRE

(30) Priority: 28.05.1999 US 322339
(43) Date of publication of application: 27.02.2002
(73) Proprietor: ABBOTT LABORATORIES, Abbott Park, IL 60064-6050 (US)
(72) Inventor: QUN, Li, Libertyville, IL 60048 (US); SHAM, Hing, Mundelein, IL 60060 (US); STEINER, Beth, A., Wooster, OH 44691-3023 (US); GWALTNEY, Stephen, L., II, Lindenhurst, IL 60046 (US); BARR, Kenneth, J., San Francisco, CA 94115 (US); IMADE, Hovis, M., Chicago, IL 60626 (US); ROSENBERG, Saul, Grayslake, IL 60030 (US); WOODS, Keith, W., Libertyville, IL 60048 (US)
(74) Representative: Modiano, Guido, Dr.-Ing.
(86) International application number: PCT/US2000/014742
(87) International publication number: WO 2000/073264

(56) References cited:
- EP-A- 0 949 238
- WO-A-98/05315
- WO-A-99/36391
- GB-A- 1 541 318
- GB-A- 1 557 622
- US-A- 5 444 036
- US-A- 5 840 991
- R.J. HALL, ET AL.: "The synthesis of pyrido[4,3-b]carbazoles from diphenyleneamine derivatives: alternative routes to and relay synthesis of ellipticines and olivacines" JOURNAL OF THE CHEMICAL SOCIETY, PERKIN TRANSACTIONS 1, no. 24, 21 December 1992 (1992-12-21), pages 3439-3450, XP002149053 Royal Society of Chemistry, Letchworth, GB ISSN: 0300-922X
- E. BACIOCCHI, ET AL.: "Oxidations of Benzyl and Phenethyl Phenyl Sulfides. Implications for the Mechanism of the Microsomal and Biomimetic Oxidation of Sulfides" TETRAHEDRON, vol. 53, no. 36, 8 September 1997 (1997-09-08), pages 12287-12298, XP004106082 Elsevier Science Publishers, Amsterdam, NL ISSN: 0040-4020
- V.V. ZAKATOV, ET AL.: "Effect of arenesulphonamide group on the nature of substitution in the aromatic ring IV. Reaction of derivatives of 4-sulphomamidophenol and 1,4-di(sulphomamido)benzene with nitrosating agents" RUSSIAN JOURNAL OF ORGANIC CHEMISTRY., vol. 30, no. 2, pt. 2, February 1994 (1994-02), pages 303-306, XP002150413 Consultants Bureau, New York, US ISSN: 1070-4280
- S. THEA, ET AL.: "The effet of leaving group variation on reactivity in the dissociative hydrolysis of aryl 3,5-dimethyl-4-hydroxybenzenesulphonate" JOURNAL OF THE CHEMICAL SOCIETY, PERKIN TRANSACTIONS 2, no. 11, November 1997 (1997-11), pages 2215-2218, XP002150414 Royal Society of Chemistry, Letchworth, GB ISSN: 0300-9580
- E. MÜLLER, ET AL.: "Dehydrierungs-Additions-Reaktionen mit Aroxylen, I. Neue radikaisch verlaufende Synthesen von Arylalkyläthern, Arylestern, Chinoläthern und p-Hydroxy-diphenyläthern" LIEBIGS ANNALEN DER CHEMIE, vol. 673, 4 May 1964 (1964-05-04), pages 40-59, XP002150415 Verlag Chemie, Weinheim, DE ISSN: 0170-2041
- H. SUZUKI, ET AL.: "Direct conversion of benzyl alcohols into benzyl sulphides with organic disulphide/diphosphorus tetraiodide" CHEMISTRY LETTERS, no. 2, February 1981 (1981-02), pages 267-268, XP002150416 Chemical Society of Japan, Tokyo, JP ISSN: 0366-7022

## Description

The present invention relates to compounds useful for treating pathological states which arise from or are exacerbated by cell proliferation and to pharmaceutical compositions comprising these compounds.

Neoplastic diseases, characterized by the proliferation of cells which are not subject to normal cell proliferating controls, are a major cause of death in humans and other mammals. Cancer chemotherapy has provided new and more effective drugs to treat these diseases and has also demonstrated that drugs which disrupt microtubule synthesis are effective in inhibiting the proliferation of neoplastic cells.

Microtubules play a key role in the regulation of cell architecture, metabolism, and division. The microtubule system of eucaryotic cells comprises a dynamic assembly and disassembly matrix in which heterodimers of tubulin polymerize to form microtubules in both normal and neoplastic cells. Within noeplastic cells, tubulin is polymerized into microtubules which form the mitotic spindle. The microtubules are then depolymerized when the mitotic spindle's use has been fulfilled. Agents which disrupt the polymerization or depolymerization of microtubules in neoplastic cells, thereby inhibiting the proliferation of these cells, comprise some of the most effective cancer chemotherapeutic agents in use.

Because of the pivotal role played by cell proliferation, agents which inhibit microtubule polymerization have been the subject of active current research for their clinical potential. See, for example, U.S. 5,767,283, U.S. 5,721,246, and U.S. 5,610,320. But there is still a need for tubulin polymerization-inhibiting compounds with modified or improved profiles of activity.

Disclosed in WO 98/05315 are pentafluorobenzenesulfonamides which are modulators of cell proliferation through binding to the ß-subunit of tubulin. Disclosed in U.S. 5,840,991 are 3,4,5-trialkoxybenzyl substituted phenyl sulfoxides useful as low density lipoprotein peroxidation inhibitors, anti-atherosclerotic agents, anti-hyperlipidemic agents and/or antihypercholesterolemic agents.

### Summary of The Invention

In one embodiment of the present invention are disclosed microtubule polymerization-inhibiting compounds represented by formula (I) 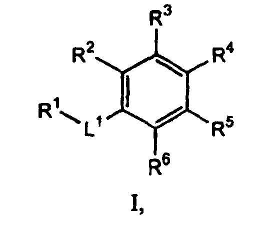 or a pharmaceutically acceptable salt thereof, wherein L¹ is selected from the group consisting of
(1) -OS(O)₂-, and
(2) -SO₂NR⁷-, wherein R⁷ is selected from the group consisting of
   (a) hydrogen,
   (b) alkanoyl,
   (c) alkyl, and
   (d) aryloyl, wherein the aryl is phenyl,
      wherein (b)-(d) can be optionally substituted with one or two substituents independently selected from the group consisting of
      (i) hydroxyl,
      (ii) halo,
      (iii) carboxy,
      (iv) phenyl,
      (v) imidazolyl,
      (vi) heterocycloalkyl selected from the group consisting of morpholinyl and piperazinyl,
      (vii) -NR^{c}R^{d}, wherein R^{c} and R^{d} are independently selected from the group consisting of
         (1') hydrogen,
         (2') alkyl,
            and
         (3') alkoxyalkyl,
            and
      (viii) -(alkylene)-NR^{c}R^{d},
      wherein for (v) and (vi), the imidazolyl and the heterocycloalkyl can be optionally substituted with one alkyl substituent and
   (e) pyrrolidinylcarbonyl wherein the pyrrolidinylcarbonyl can be optionally substituted with one alkyl substituent
wherein (1)-(2) are shown with their left ends attached to R¹ and their right ends attached to the phenyl ring;
R¹ is heteroaryl, wherein the heteroaryl is selected from the group consisting of indolinyl and indolyl, and wherein the heteroaryl can be optionally substituted with one or two substituents independently selected from the group consisting of
(a) carboxaldehyde,
(b) nitro,
(c) alkyl, and
(d) -NH₂;
R² and R⁶ are hydrogen;
and
R³, R⁴, and R⁵ are alkoxy.

In a preferred embodiment of the invention are compounds wherein L¹ is -SO₂NR⁷-, and R⁷ is defined above.

In another preferred embodiment of the invention are compounds wherein R³, R⁴ and R⁵ are methoxy, particularly when R¹ is optionally substituted 2,3-dihydro-1H-indol-5-yl, optionally substituted 1H-indol-3-yl or optionally substituted 1H-indol-5-yl.

In another preferred embodiment of the invention are compounds wherein R¹ is N-methyl substituted 1H-indolyl.

In another preferred embodiment of the invention are compounds wherein R⁷ is substituted alkanoyl, substituted aryloyl wherein the aryl is phenyl, or optionally substituted pyrrolidinylcarbonyl.

In yet another preferred embodiment of the invention are compounds wherein L¹ is -OSO₂-.

The compounds of the invention can be used in methods of inhibiting polymerization of tubulin in a mammal in recognized need of such treatment comprising administering an effective amount of a compound having formula (I).

The compounds of the invention can also be used in methods of treating cancer in a mammal in recognized need of such treatment comprising administering an effective amount of a compound having formula (I).

In still yet another embodiment of the invention are disclosed pharmaceutical compositions containing compounds having formula (I).

### Detailed Description of The Invention

### Definition of Terms

The term "alkanoyl," as used herein, refers to an alkyl group attached to the parent molecular group through a carbonyl group.

The term "alkoxy," as used herein, refers to an alkyl group attached to the parent molecular group through an oxygen atom.

The term "alkoxyalkyl," as used herein, refers to an alkoxy group attached to the parent molecular moiety through an alkyl group.

The term "alkyl," as used herein, refers to a monovalent group of one to six carbon atoms derived from a straight or branched chain saturated hydrocarbon.

The term "alkylating agent," as used herein, represents a reagent capable of donating an alkyl group during the course of a reaction. Examples of alkylating agents include methyl triflate, dimethyl sulfate, iodomethane, bromobutane, bromopropane, and the like.

The term "alkylene," as used herein, refers to a saturated divalent hydrocarbon group derived from a straight or branched chain saturated hydrocarbon by the removal of two hydrogen atoms.

The term "aryloyl," as used herein, refers to an aryl group attached to the parent molecular moiety through a carbonyl group.

The term "azido," as used herein, refers to -N₃.

The term "base," as used herein, represents a reagent capable of accepting protons during the course of a reaction. Examples of bases include carbonates such as potassium carbonate, potassium bicarbonate sodium carbonate, sodium bicarbonate, and cesium carbonate; halides such as cesium fluoride; phosphates such as potassium phosphate, potassium dihydrogen phosphate, and potassium hydrogen phosphate; hydroxides such as lithium hydroxide, sodium hydroxide, and potassium hydroxide; disilylamides such as lithium hexamethyldisilazide, potassium hexamethyldisilazide, and sodium hexamethyldisilazide; trialkylamines such as triethylamine and diisopropylamine; heterocyclic amines such as imidazole, pyridine, pyridazine, pyrimidine, and pyrazine; bicyclic amines such as DBN and DBU; and hydrides such as lithium hydride, sodium hydride, and potassium hydride. The base chosen for a particular conversion depends on the nature of the starting materials, the solvent or solvents in which the reaction is conducted, and the temperature at which the reaction is conducted.

The term "carboxaldehyde," as used herein, refers to -CHO.

The term "carbonyl," as used herein, refers to -C(O)-.

The term "carboxy," as used herein, refers to -CO₂H.

The term "halo," as used herein, refers to -F, -Cl, -Br or -I.

The term "hydroxy," as used herein, refers to -OH.

The term "nitro," as used herein, refers to -NO₂.

The term "nitrogen-protecting group," as used herein, refers to groups intended to protect an amino group against undesirable reactions during synthetic procedures. Commonly used nitrogen-protecting groups are disclosed in Greene, "Protective Groups In Organic Synthesis," (John Wiley & Sons, New York (1991)). Common N-protecting groups comprise (a) acyl groups such as formyl, acetyl, propionyl, pivaloyl, *tert*-butylacetyl, 2-chloroacetyl, 2-bromoacetyl, trifluoroacetyl, trichloroacetyl, phthalyl, *o*-nitrophenoxyacetyl, α-chlorobutyryl, benzoyl, 4-chlorobenzoyl, 4-bromobenzoyl, and 4-nitrobenzoyl, (b) sulfonyl groups such as benzenesulfonyl, and *para*-toluenesulfonyl, (c) carbamate forming groups such as benzyloxycarbonyl, *para*-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, *tert*-butyloxycarbonyl, diisopropylmethoxycarbonyl, isopropyloxycarbonyl, ethoxycarbonyl, methoxycarbonyl, allyloxycarbonyl, 2,2,2-trichloroethoxycarbonyl, phenoxycarbonyl, 4-nitrophenoxy carbonyl, cyclopentyloxycarbonyl, adamantyloxycarbonyl, cyclohexyloxycarbonyl, and phenylthiocarbonyl, (d) arylalkyl groups such as benzyl, triphenylmethyl, and benzyloxymethyl, and (e) silyl groups such as trimethylsily. Preferred N-protecting groups are formyl, acetyl, benzoyl, pivaloyl, *tert*-butylacetyl, phenylsulfonyl, benzyl, *tert*-butyloxycarbonyl (Boc) and benzyloxycarbonyl (Cbz).

The term "pharmaceutically acceptable salt," as used herein, refers to salts which are, within the scope of sound medical judgment, suitable for use in contact with the tissues of humans and lower animals without undue toxicity, irritation, or allergic response and are commensurate with a reasonable benefit/risk ratio. Pharmaceutically acceptable salts are well-known in the art. For example, S. M. Berge, *et al.* describe pharmaceutically acceptable salts in detail in J. Pharmaceutical Sciences, 1977, 66: 1 *et seq.* The salts may be prepared *in situ* during the final isolation and purification of the compounds of the invention or separately by reacting a free base function with a suitable acid. Representative acid addition salts include acetate, adipate, alginate, citrate, aspartate, benzoate, benzenesulfonate, bisulfate, butyrate, camphorate, camphorsufonate, digluconate, glycerophosphate, hemisulfate, heptanoate, hexanoate, fumarate, hydrochloride, hydrobromide, hydroiodide, 2-hydroxyethansulfonate (isethionate), lactate, maleate, methanesulfonate, nicotinate, 2-naphthalenesulfonate, oxalate, parnoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates such as dimethyl, diethyl, dibutyl and diamyl sulfates; long chain halides such as decyl, lauryl, myristyl, and stearyl chlorides, bromides and iodides; and arylalkyl halides such as benzyl and phenethyl bromides. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid, and citric acid.

Basic addition salts can be prepared *in situ* during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, and ethylamine. Other representative organic amines useful for the formation of base addition salts include ethylenediamine, ethanolamine, diethanolamine, piperidine, and piperazine.

The compounds of the invention may be administered as pharmaceutically acceptable prodrugs which are those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention.

The term "prodrug," as used herein, represents compounds which are rapidly transformed *in vivo* to parent compounds having formula (I), for example, by hydrolysis in blood. A thorough discussion is provided in T. Higuchi and V. Stella, Prodrugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987. Particularly preferred prodrugs include compounds having formula (I), wherein a nitrogen or hydroxy group has attached thereto an aminoacyl, bisaminoacyl (2-mer), or trisaminoacyl (3-mer) group optionally capped with a carboxyl protecting group. The term "aminoacyl," as used herein, refers to a group derived from naturally or unnaturally occuring amino acids. Representative aminoacyl groups include those derived from glycine, alanine, β-alanine, valine, leucine, *iso*-leucine, methionine, serine, threonine, cysteine, phenylalanine, and tyrosine in the racemic, D or L configurations. The aminoacyl groups of this invention can be optionally substituted. The terms "bisaminoacyl" and "trisaminoacyl," as used herein, refer to di- and tri- aminoacyl groups, respectively. Representative examples of bisaminoacyl and trisaminoacyl groups include 2-mers and 3-mers derived from glycine, alanine, β-alanine, valine, leucine, *iso*-leucine, methionine, serine, threonine, cysteine, phenylalanine, and tyrosine in the racemic, D or L configurations.

The compounds of the present invention may form metabolites by *in vivo* biotransformation of compounds having formula (I). The term "metabolite," as used herein, refers to compounds formed by *in vivo* biotransformation of compounds having formula (I) by oxidation, reduction, hydrolysis, or conjugation. Other compounds may undergo *in vivo* biotransformation such as by oxidation, reduction, hydrolysis, or conjugation to form compounds having formula (I). A thorough discussion of biotransformation is provided in Goodman and Gilman's, The Pharmacological Basis of Therapeutics, seventh edition.

Asymmetric or chiral centers may exist in the compounds of the present invention. The present invention contemplates the various stereoisomers and mixtures thereof. Individual stereoisomers of compounds of the present invention are prepared synthetically from commercially available starting materials which contain asymmetric or chiral centers or by preparation of mixtures of enantiomeric compounds followed by resolution well-known to those of ordinary skill in the art. These methods of resolution are exemplified by (1) attachment of a racemic mixture of enantiomers to a chiral auxiliary, separation of the resulting diastereomers by recrystallization or chromatography and liberation of the optically pure product from the auxiliary or (2) direct separation of the mixture of optical enantiomers on chiral chromatographic columns.

Geometric isomers may also exist in the compounds of the present invention. The present invention contemplates the various geometric isomers and mixtures thereof resulting from the arrangement of substituents around a carbon-carbon double bond.

Compounds falling within the scope of formula (I) include, but are not limited to
1-formyl-*N*-(3,4,5-trimethoxyphenyl)indoline-5-sulfonamide,
*N*-(3,4,5-trimethoxyphenyl)indoline-5-sulfonamide;
5-nitro-*N*-(3,4,5-trimethoxyphenyl)-1*H*-indole-3-sulfonamide,
1-methyl-*N*-(3,4,5-trimethoxyphenyl)indoline-5-sulfonamide,
1-methyl-5-nitro-*N*-(3,4,5-trimethoxyphenyl)-1*H*-indole-3-sulfonamide,
5-amino-1-methyl-*N*-(3,4,5-trimethoxyphenyl)-1*H*-indole-3-sulfonamide,
5-amino-*N*-(3,4,5-trimethoxyphenyl)-1*H*-indole-3-sulfonamide,
*N*-(3,4,5-trimethoxyphenyl)-1*H*-indole-5-sulfonamide,
1-methyl-*N*-(3,4,5-trimethoxyphenyl)-1*H*-indole-5-sulfonamide,
*N*,1-dimethyl-N-(3,4,5-trimethoxyphenyl)-1*H*-indole-5-sulfonamide,
1*H*-indol-5-yl 3,4,5-trimethoxybenzenesulfonate,
1-methyl-1*H*-indol-5-yl 3,4,5-trimethoxybenzenesulfonate,
1-ethyl-*N*-(3,4,5-trimethoxyphenyl)-1*H*-indole-5-sulfonamide,
3,4,5-trimethoxy-*N*-(6-quinolinyl)benzenesulfonamide,
*N*-(2-hydroxyethyl)-1-methyl-*N*-(3,4,5-trimethoxyphenyl)-1*H*-indole-5-sulfonamide,
*N*-(2-fluoroethyl)-1-methyl-*N*-(3,4,5-trimethoxyphenyl)-1*H*-indole-5-sulfonamide,
*N*-ethyl-1-methyl-*N*-(3,4,5-trimethoxyphenyl)-1*H*-indole-5-sulfonamide,
*N*-((dimethylamino)acetyl)-1-methyl-*N*-(3,4,5-trimethoxyphenyl)-1*H*-indole-5-sulfonamide,
1-methyl-*N*-(((2S)-1-methylpyrrolidinyl)carbonyl)-*N*-(3,4,5-trimethoxyphenyl)-1*H*-indole-5-sulfonamide,
*N*-((2*S*)-2-(dimethylamino)-3-methylbutanoyl)-1-methyl-*N*-(3,4,5-trimethoxyphenyl)-1*H*-indole-5-sulfonamide,
*N*-((2*S*)-2-amino-3-methylbutanoyl)-1-methyl-*N*-(3,4,5-trimethoxyphenyl)-1*H*-indole-5-sulfonamide,
1-methyl-*N*-((2*S*)-2-methylamino)propanoyl)-*N*-(3,4,5-trimethoxyphenyl)-1*H*-indole-5-sulfonamide,
*N*-((2*S*)-2-amino-2-phenylethanoyl)-1-methyl-*N*-(3,4,5-trimethoxyphenyl)-1*H*-indole-5-sulfonamide,
*N*-((2*S*)-2-amino-3-phenylpropanoyl)-1-methyl-*N*-(3,4,5-trimethoxyphenyl)-1*H*-indole-5-sulfonamide,
1-methyl-*N*-((2*S*)-pyrrolidinylcarbonyl)-*N*-(3,4,5-trimethoxyphenyl)-1*H*-indole-5-sulfonamide,
*N*-((2*S*)-2,6-diaminohexanoyl)-1-methyl-*N*-(3,4,5-trimethoxyphenyl)-1*H*-indole-5-sulfonamide,
*N*-((2*S*)-2-amino-3-(1*H*-imidazol-5-yl)propanoyl)-1-methyl-*N*-(3,4,5-trimethoxyphenyl)-1*H*-indole-5-sulfonamide,
(2*S*)-2-amino-4-oxo-4-(3,4,5-trimethoxy((1-methyl-1*H*-indol-5-yl)sulfonyl)anilino)butanoic acid,
(3*S*)-3-amino-4-oxo-4-(3,4,5-trimethoxy((1-methyl-1*H*-indol-5-yl)sulfonyl)anilino)butanoic acid,
(2*S*)-2-amino-5-oxo-5-(3,4,5-thrimethoxy((1-methyl-1*H*-indol-5-yl)sulfonyl)anilino)pentanoic acid,
(4*S*)-4-amino-5-oxo-5-(3,4,5-trimethoxy((1-methyl-1*H*-indol-5-yl)sulfonyl)anilino)pentanoic acid,
*N*-((bis(2-methoxyethyl)amino)acetyl)-1-methyl-*N*-(3,4,5-trimethoxyphenyl)-1*H*-indole-5-sulfonamide,
1-methyl-*N*-(4-morpholinylacetyl)-*N*-(3,4,5-trimethoxyphenyl)-1*H*-indole-5-sulfonamide,
1-methyl-*N*-((4-methyl-1-piperazinyl)acetyl)-*N*-(3,4,5-trimethoxyphenyl)-1*H*-indole-5-sulfonamide,
*N*-(4-(aminomethyl)benzoyl)-1-methyl-*N*-(3,4,5-trimethoxyphenyl)-1*H*-indole-5-sulfonamide,
*N*-(2-aminoacetyl)-1-methyl-*N*-(3,4,5-trimethoxyphenyl)-1*H*-indole-5-sulfonamide,
*N*-((2*S*)-2-aminopropanoyl)-1-methyl-*N*-(3,4,5-trimethoxyphenyl)-1*H*-indole-5-sulfonamide,
*N*-(3-aminopropanoyl)-1-methyl-*N*-(3,4,5-trimethoxyphenyl)-1*H*-indole-5-sulfonamide,
(2*S*)-2-amino-*N*-((1*S*)-1-methyl-2-oxo-2-(3,4,5-trimethoxy((1-methyl-1*H*-indol-5-yl)sulfonyl)anilino)ethyl)propanamide, and
*N*-((2*S*)-2-amino-3-hydroxypropanoyl)-1-methyl-*N*-(3,4,5-trimethoxyphenyl)-1*H*-indole-5-sulfonamide.

A more preferred compound for the practice of the present invention is *N*-((dimethylamino)acetyl)-1-methyl-*N*-(3,4,5-trimethoxyphenyl)-1*H*-indole-5-sulfonamide.

### Determinatian of Biological Activity

Compounds of this invention were tested in a 48-hour cellular proliferation assay which uses human colon adenocarcinoma, MDR positive (HCT-15) cells, and human lung large cell carcinoma, MDR negative (NCI-H460) cells, in the 96-well microtitre format described in Skehan P., et al. New Colorimetric Cytotoxicity Assay for Anticancer Drug Screening. 1990, J. Natl. Cancer Inst. 82:1107-1112. Briefly, the wells of a microtitre plate were charged sequentially with cultured cells and compounds of the invention (1.0 x 10⁻⁴ to 1.0 x 10⁻¹¹ M in 10% DMSO prepared by dissolving compounds of the invention in DMSO and adding 11 µL of the DMSO solution to 100 µL of culture medium for a final DMSO concentration of 10%). Two of the following controls were also present in each microtitre plate: a solvent (DMSO) control without drug that yielded a 0% inhibition level and a trichloroacetic acid-treated well that yielded a 100% inhibition level. The cells were grown in culture (37 °C, 5% CO₂ atmosphere) for 48 hours then fixed by the addition of trichloroacetic acid. The wells were stained with sulforhodamine, washed with 1% acetic acid, and treated with 0.01 M tris buffer (100 µL) to solubilize the adherent dye. The absorbance of the dye solution was measured with a Molecular Devices SpechaMax340 plate reader. The percent inhibition values were obtained by calculating the proportional response of the experimental values to the absorbance values of the controls. The results for representative examples of compounds having formula (I) are shown in Table 1.

**Table 1**

| Inhibitory Potency of Representative Compounds | | |
|---|---|---|
| Example | NCI-460 % inhibition at 10⁻⁴M | HCT-15 % inhibition at 10⁻⁴M |
| 1 | 98.5 | 99.4 |
| 2 | 74.0 | 79.9 |
| 3 | 59.3 | 86.2 |
| 4 | 41.7 | 15.9 |
| 5 | 96.1 | 97.6 |
| 6 | 45.5 | 50.6 |
| 7 | 87.0 | 91.9 |
| 8 | <9.1 | 37.0 |
| 9 | 89.1 | 94.1 |
| 10 | 100.0 | 100.0 |
| 11 | 99.9 | 100.0 |
| 15 | 100.0 | 100.0 |
| 16 | 100.0 | 100.0 |

As shown by the data in Table 1, the compounds of the invention, including, but not limited to, those specified in the examples, are useful for the treatment of disease caused or exascerbated by cell proliferation. As cell proliferation inhibitors, these compounds are useful in the treatment of both primary and metastatic solid tumors and carcinomas of the breast, colon, rectum, lung, oropharynx, hypopharynx, esophagus, stomach, pancreas, liver, gallbladder, bile ducts, small intestine, urinary tract including kidney, bladder and urothelium, female genital tract including cervix, uterus, ovaries, choriocarcinoma, and gestational trophoblastic disease, male genital tract including prostate, seminal vesicles, testes, and germ cell tumors, endocrine glands including thyroid, adrenal, and pituitary, skin including hemangiomas, melanomas, sarcomas arising from bone or soft tissues including Kaposi's sarcoma, tumors of the brain, nerves, and eyes, meninges including astrocytomas, gliomas, glioblastomas, retinoblastomas, neuromas, neuroblastomas, Schwannomas and meningiomas, solid tumors arising from hematopoietic malignancies including leukemias and chloromas, plasmacytomas, plaques, tumors of mycosis fungoides, cutaneous T-cell lymphoma/leukemia, lymphomas including Hodgkin's and non-Hodgkin's lymphomas, prophylaxis of autoimmune diseases including rheumatoid, immune and degenerative arthritis, ocular diseases including diabetic retinopathy, retinopathy of prematurity, corneal graft rejection, retrolental fibroplasia, neovascular glaucoma, rubeosis, retinal neovascularization due to macular degeneration, hypoxia, abnormal neovascularization conditions of the eye, skin diseases including psoriasis, blood vessel diseases including hemagiomas and capillary proliferation within atherosclerotic plaques, Osler-Webber Syndrome, myocardial angiogenesis, plaque neovascularization, telangiectasia, hemophiliac joints, angiofibroma, and wound granulation.

The compounds of the present invention may also be useful for the prevention of metastases from the tumors described above either when used alone or in combination with radiotherapy and/or other chemotherapeutic treatments conventionally administered to patients for treating cancer. For example, when used in the treatment of solid tumors, compounds of the present invention may be administered with chemotherapeutic agents such as alpha inteferon, COMP (cyclophosphamide, vincristine, methotrexate, and prednisone), etoposide, mBACOD (methortrexate, bleomycin, doxorubicin, cyclophosphamide, vincristine, and dexamethasone), PRO-MACE/MOPP (prednisone, methotrexate (w/leucovin rescue), doxorubicin, cyclophosphamide, paclitaxel, etoposide/mechlorethamine, vincristine, prednisone, and procarbazine), vincristine, vinblastine, angioinhibins, TNP-470, pentosan polysulfate, platelet factor 4, angiostatin, LM-609, SU-101, CM-101, Techgalan, thalidomide, SP-PG, and the like. Other chemotherapeutic agents include alkylating agents such as nitrogen mustards (mechloethamine, melphan, chlorambucil, cyclophosphamide and ifosfamide), nitrosoureas including carmustine, lomustine, semustine and streptozocin, alkyl sulfonates including busulfan, triazines including dacarbazine, ethyenimines including thiotepa and hexamethylmelamine, folic acid analogs including methotrexate, pyrimidine analogues including 5-fluorouracil and cytosine arabinoside, purine analogs including 6-mercaptopurine and 6-thioguanine, antitumor antibiotics including actinomycin D, anthracyclines including doxorubicin, bleomycin, mitomycin C and methramycin, hormones and hormone antagonists including tamoxifen, cortiosteroids and miscellaneous agents including cisplatin and brequinar. For example, a tumor may be treated conventionally with surgery, radiation, or chemotherapy, and compounds having formula (I), then treated with additional compound having formula (I) to extend the dormancy of micrometastases and to stabilize and inhibit the growth of any residual primary tumor.

### Methods of Treatment

The present invention also provides pharmaceutical compositions which comprise compounds of the present invention formulated together with one or more non-toxic pharmaceutically acceptable carriers. The pharmaceutical compositions may be specially formulated for oral administration in solid or liquid form, for parenteral injection, or for rectal administration.

The pharmaceutical compositions of this invention can be administered to humans and other animals orally, rectally, parenterally, intracisternally, intravaginally, intraperitoneally, topically (as by powders, ointments, or drops), bucally, or as an oral or nasal spray. The term "parenteral" administration as used herein refers to modes of administration which include intravenous, intramuscular, intraperitoneal, intrasternal, subcutaneous and intraarticular injection and infusion.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like, Prolonged absorption of the injectable pharmaceutical form may be brought about by the inclusion of agents which delay absorption such as aluminum monostearate and gelatin.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1;3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

Compositions for rectal or vaginal administration are preferably suppositories which can be prepared by mixing the compounds of this invention with suitable nonirritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The compositions in liposome form can contain, in addition to a compound of the present invention, stabilizers, preservatives, excipients, and the like. The preferred lipids are the phospholipids and the phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 *et seq.*

Dosage forms for topical administration of a compound of this invention include powders, sprays, ointments and inhalants. The active compound is mixed under sterile conditions with a pharmaceutically acceptable carrier and any needed preservatives, buffers, or propellants which may be required. Opthalmic formulations, eye ointments, powders and solutions are also contemplated as being within the scope of this invention. Actual dosage levels of active ingredients in the pharmaceutical compositions of this invention may be varied so as to obtain an amount of the active compound(s) that is effective to achieve the desired therapeutic response for a particular patient, compositions, and mode of administration. The selected dosage level will depend upon the activity of the particular compound, the route of administration, the severity of the condition being treated, and the condition and prior medical history of the patient being treated. However, it is within the skill of the art to start doses of the compound at levels lower than required for to achieve the desired therapeutic effect and to gradually increase the dosage until the desired effect is achieved.

Generally dosage levels of about 1 to about 50, more preferably of about 5 to about 20 mg of active compound per kilogram of body weight per day are administered orally to a mammalian patient. If desired, the effective daily dose may be divided into multiple doses for purposes of administration, e.g. two to four separate doses per day.

### Synthetic Methods

The compounds of the present invention will be better understood in connection with the following synthetic schemes, which illustrate methods by which the compounds of the invention may be prepared. The compounds having formula (I) may be prepared by a variety of synthetic routes. Representative procedures are shown in Scheme 1. The groups R¹, R², R³, R⁴, R⁵, R⁶, and L¹, are as previously defined unless otherwise noted. It will be readily apparent to one of ordinary skill in the art that other compounds within formula (I) can be synthesized by substitution of the appropriate reactants and agents in the syntheses shown below. It will further be apparent to one skilled in the art that the selective protection and deprotection steps, as well as order of the steps themselves, can be carried out in varying order, depending on the nature of groups R¹, R², R³, R⁴, R⁵, R⁶ and L¹, to successfully complete the syntheses of compounds having formula (I). Commonly used protecting groups are disclosed in Greene, "Protective Groups In Organic Synthesis," John Wiley & Sons, New York (1981). It will still further be apparent to one of ordinary skill in the art that the substituents R¹, R², R³, R⁴, R⁵, R⁶ and L¹ can be determined by selection of the appropriate commercially available or known starting materials or introduced synthetically by known chemical methods such as those disclosed in Larock, "Comprehensive Organic Transformations. A Guide to Functional Group Preparations," VCH Publishers, New York (1989).

### Abbreviations

Abbreviations used in the descriptions of the schemes the examples are: THF for tetrahydrofuran; DMF for N,N-dimethylformamide; DMSO for dimethylsulfoxide; DEAD for diethyl azodicarboxylate; DIAD for diisopropyl azodicarboxylate; EDC for 1-(3-dimethylaminopropyl)-3-ethylcarbodiimide hydrochloride; LDA for lithium diisopropylamide; TFA for trifluoroacetic acid; DMSO for dimethylsulfoxide; DMAP for 4-(N,N-dimethylamino)pyridine; HATU for O-(azabenzotriazole-1-yl)-N,N,N',N'-tetramethyluroniumhexafluorophosphate; Boc for *tert-*butylcarbonyloxy; DPPA for diphenylphosphoryl azide; DCC for dicyclohexylcarbodiimide; HOOBT for 3-hydroxy-1,2,3-benzotriazin-4(3*H*)-one; HOBT for 1-hydroxybenzotriazole hydrate; EDCI for 1-(3-dimethylaminopropyl)-3-ethylcarbodiimide hydrochloride; CDI for 1,1'-carbonyldiimidazole; and DMAP for N,N-dimethylaminopyridine.

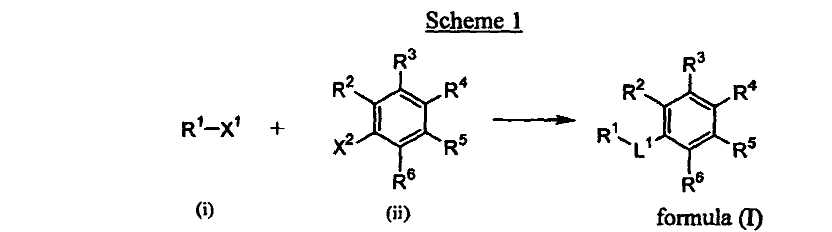

As shown in Scheme 1, the compounds having formula (I) were prepared by reacting intermediate (i) with intermediate (ii), wherein X¹ and X² together are L¹. With either (i) or (ii), X¹ or X² can be any conventional activated sulfonic acid, examples of which include sulfonyl halides, sulfonic acid anhydrides, and *N*-sulfonylimidazolides, preferably sulfonyl halides. Although the solvent used in the coupling reactions is not particularly limited, a solvent in which the starting materials are both soluble and which is little reactive with the materials is preferably used. Examples of such solvents are pyridine, triethylamine, THF, dioxane, benzene, toluene, diethyl ether, dichloromethane, DMF, DMSO, or mixtures thereof. When an acid is liberated with the progress of a reaction, such as when using a halide derivative of a sulfonic acid and an amine or alcohol, it is preferable that the reaction is carried out in the presence of a suitable deacidifying agent. For this reason, the use of a basic solvent such as pyridine or triethylamine is particularly preferred, although the reaction can be run in any of the aformentioned solvents with at least a stoichiometric amount of basic solvent present. Although the reactions generally proceed at room temperature, they can be run at lower or elevated temperatures, as needed. The reaction time is generally 30 minutes to 18 hours and can be arbitrarily selected depending on the types of starting materials and reaction temperature. When the product has a protected amino or hydroxyl group, the product, if necessary, can be converted to a compound having formula (I) having a free amino or hydroxyl group by a conventional deprotection method such as treatment with acid, piperidine, or catalytic hydrogenation in the presence of a catalyst such as palladium on carbon. When the compound having formula (I) has a nitro group, the nitro group can also be reduced. Although the reduction can be conducted by any conventional process, the conversion of a nitro group to an amine is preferably conducted by catalytic hydrogenation using palladium on carbon or platinum oxide as the catalyst or reduction using an acid together with zinc, iron, or tin. The catalytic reduction is conducted in an organic solvent such as methanol, ethanol, or THF under normal or elevated temperature. Groups on the compounds having formula (I) having endogenous or exogenous amino groups can optionally alkylated, formylated, acetylated or otherwise reacted with any number of amine-derivatization reagents well-known to those of ordinary skill in the art. For example, acidic N-H groups can be reacted with alcohols under Mitsunobu conditions. Preferable Mitsunobu conditions include reacting the compounds having formula (I) with alcohols in the presence of a phosphine, preferably triphenylphosphine or tri *n*-butylphosphine and an activating agent such as DEAD or DIAD. Although the solvent to be used in the reaction is not particularly limited, polar, aprotic solvents such as THF or dioxane are particularly preferable for Mitsunobu reactions. The compounds having formula (I) can also be alkylated with any number of reagents well-known to those of ordinary skill in the art. For example, compounds having formula (I) can be reacted with an unsubstituted or substituted alkylating agent in the presence of a non-nucleophilic base such as sodium or potassium hydride or lithium, sodium, or potassium bis(trimethylsilyl)amide. Although the solvent to be used in the reaction is not particularly limited, polar, aprotic solvents such as THF, DMF, DMSO, or dioxane are particularly preferable for alkylation reactions. Compounds having formula (I) can be reacted with halogenation agents. Examples of halogenating agents include *N*-chlorosuccinamide, *N*-bromosuccinamide, 1,3-bibromo-5,5-dimethylhydantoin, *N*-bromoacetamide, bromine, chlorine, or iodine. Although the solvent to be used in the reaction is not particularly limited, chloroalkanes such as dichloromethane, chloroform, or carbon tetrachloride, halogenated aromatic rings such as chlorobenzene and dichlorobenzene, water, or organic acids, such as acetic acid, are particularly preferable.

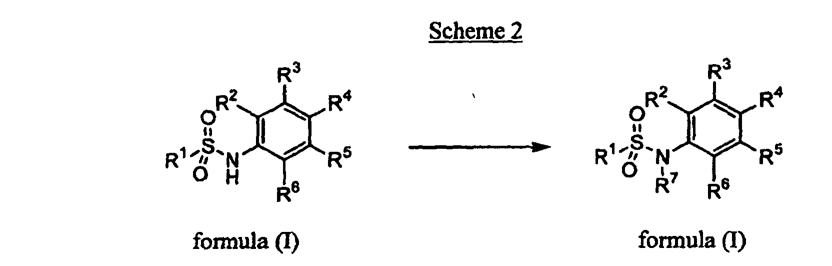

As shown in Scheme 2, compounds of formula (I) (R⁷ is H) can be intraconverted to prodrugs of compounds of formula (1) (R⁷ is an aminoacyl, bisaminoacyl (2-mer), or trisaminoacyl (3-mer) residue optionally capped with a carboxyl protecting group) by reaction with naturally or unnaturally occurring amino acids or with 2-mers and 3-mers derived from amino acids. Representative amino acids include *N*,*N*-dimethylglycine, *N*-methyl-L-proline, *N*,*N*-dimethyl-L-valine, *N*-*tert*-butoxycarbonyl)-L-valine, *N-*(*tert*-butoxy-carbonyl)-L-*N*-methylalanine, (*S*)-*N*-(*tert*-butoxycarbonyl)-2-phenylglycine, *N*-(*tert*-butoxycarbonyl)-L-phenylalanine, *N*-(*tert*-butoxycarbonyl)-L-proline, *N,N*-di-(*tert*-butoxycarbonyl)-L-lysine, *N*-(*tert*-butoxycarbonyl)-L-valine, *N*-(*tert*-butoxycarbonyl)-L-aspartic acid 1-*tert*-butyl ester, *N*-(*tert*-butoxycarbonyl)-L-aspartic acid 4-tert-butyl ester, *N*-(*tert*-butoxycarbonyl)-L-glutamic acid 1-*tert*-butyl ester, *N*-(*tert*-butoxycarbonyl)-L-glutamic acid 5-*tert*-butyl ester, (bis(2-methoxyethyl)amino)acetic acid, 4-morpholinylacetic acid, (4-methyl-1-piperazinyl)acetic acid, and 4-(((*tert*-butoxy-carbonyl)amino)methyl)benzoic acid in the presence of base and an activating agent. Naturally occurring amino acids can be purchased commercially, while unnaturally occurring amino acids can be synthesized by methods well-known in the art. Representative bases include 4-pyrrolidinylpyridine, DMAP, and triethylamine. Examples of activating used in these reactions include DCC, EDCI, HOBT, and CDI. Typical solvents used in these reactions include dichloromethane, carbon tetrachloride, and chloroform. The reaction temperature is 0 °C to 30 °C and depends on the method chosen. Reaction times are typically 2 to 24 hours. In a preferred embodiment, compounds of formula (I) (R is H) in dichloromethane at room temperature are reacted with a naturally or unnaturally occurring amino acid in the presence of DCC and 4-pyrrolidinylpyridine for 16 hours to provide compounds of formula (1) (R⁷ is an aminoacyl, bisaminoacyl (2-mer), or trisaminoacyl (3-mer) residue optionally capped with a carboxyl protecting group).

The compounds and processes of the present invention will be better understood in connection with the following examples.

### Example 1

### 4-methoxy-N-(3,4,5-trimethoxyphenyl)benzenesulfonamide

A solution of 3,4,5-trimethoxyaniline (500 mg, 2.8 mmol) in pyridine (5 mL) was treated with 4-methoxybenzenesulfonyl chloride (564 mg, 2.8 mmol) in THF (5 mL), stirred at room temperature for 18 hours, concentrated, redissolved in THF (1 mL), treated with water with stirring, and filtered to provide 820 mg of the desired product.
MS (DCI/NH₃) *m*/*z* 354 (M+H)⁺ and 371 (M+NH₄)⁺;
¹H NMR (300 MHz, CDCl₃) δ 7.85 (d, J=7.5 Hz, 2H), 6.91 (d, J=7.5 Hz, 2H), 6.29 (s, 2H), 3.84 (s, 3H), 3.78 (s, 3H), 3.75 (s, 6H).

### Example 2

### 1-formyl-N-(3,4,5-trimethoxyphenyl)indoline-5-sulfonamide

### Example 2A

### N-formylindoline

A solution of indoline (5.0 g) and 98% formic acid (3.0 g) in toluene (17 mL) was heated at reflux for 6 hours with a Dean-Stark trap, cooled, washed with water and concentrated. The resulting dark brown solid was dissolved in methanol, concentrated to a fraction of its original volume, treated with 1: diethyl ether/hexane, and concentrated dryness to provide 5.5 g of the desired product.

### Example 2B

### 5-chlorosulfonylindoline-1-carboxaldehyde

Chlorosulfonic acid (4.6 mL) at 0 °C was treated portionwise with a sample of Example 2A (2.0 g) over 30 minutes, stirred for 5 minutes at 0 °C, heated at 100 °C until all bubbling ceased, carefully poured over ice and water, stired vigorously for 2 hours, filtered, and dried overnight in a vacuum oven to provide 2.5 g of the desired product.

### Example 2C

### 1-formyl-N-(3,4,5-trimethoxyphenyl)indoline-5-sulfonamide

3,4,5-trimethoxyaniline (2.51 g) was processed as described in Example 1 (substituting Example 2B for 4-methoxybenzenesulfonyl chloride) and purified by column chromatography on silica gel with 2% methanol/dichloromethane to provide 4.8 g of the desired product.
MS (DCI/NH₃) 410 (M+NH₄)⁺;
¹H NMR (DMSO-d₆, 3 00 MHz) δ 9.85 (s, 1H), 7.05 (d, J=2.4 Hz, 1H), 6.98 (dd, J=2.4, 8.1 Hz, 1H), 6.88 (d, J=8.1 Hz, 1H), 6.38 (s, 2H), 5.19 (s, 1H), 3.80 (s, 3H), 3.65 (s, 6H), 3.55 (s, 3H).

### Example 3

### N-(3,4,5-trimethoxyphenyl)indoline-5-sulfonamide

A solution of Example 2C (4.8 g) in methanol (60 mL) at room temperature was treated with HCl gas for about 8 minutes, concentrated to dryness, and treated with ethyl acetate and water. The organic layer was washed with saturated aqueous NaHCO₃ until the aqueous washings were slightly basic, dried (Na₂SO₄) filtered, and concentrated to provide 4.0 g of the desired product.
MS (DCI/NH₃) *m*/*z* 365 (M+H)⁺ 382 (M+NH₄)⁺;
¹H NMR (DMSO-d₆, 300 MHz) δ 9.69 (s, 1H), 7.34 (m, 1H), 6.42 (m, 2H), 6.37 (s, 3H), 3.65 (s, 6H), 3.53 (s, 3H), 3.50 (t, J=9 Hz, 2H), 2.94 (t, J=9 Hz, 2H).

### Example 4

### 5-nitro-N-(3,4,5-trimethoxyphenyl)-1H-indole-3-sulfonamide

### Example 4A

### 5-nitro-1H-indole-3-sulfonyl chloride

A solution of chlorosulfonic acid (3 mL, 45 mmol) and Na₂SO₄ (700 mg, 4.9 mmol) in dichloromethane (30 mL) was treated dropwise with a solution of 5-nitroindole (800 mg, 4.9 mmol) in dichloromethane (20 mL) over 1 hour, stirred for another 30 minures, and decanted to provide a thick brown oil. The oil was slowly treated with water (20 mL), stirred for 10 minutes, filtered, and dried in a vacuum oven to provide 651 mg of the desired product.

### Example 4B

### 5-nitro-N-(3,4,5-trimethoxyphenyl)-1H-indole-3-sulfonamide

3,4,5-trimethoxyaniline (100 mg) was processed as described in Example 1 (substituting Example 4A for 4-methoxybenzenesulfonyl chloride) and purified by column chromatography on silica gel with 2% methanol/dichloromethane to provide 120 mg of the desired product.
MS (DCI/NH₃) *m*/*z* 407 (M+H)⁺ 425 (M+NH₄)⁺;
¹H NMR (DMSO-d₆, 300 MHz) δ 10.15 (s, 1H), 8.71 (d, J=2.1 Hz, 1H), 8.27 (s, 1H), 8.10 (dd, J=2.1, 9 Hz, 1H), 7.66 (d, J=9 Hz, 1H), 6.38 (s, 2H), 3.60 (s, 6H), 3.50 (s, 3H).

### Example 5

### 1-methyl-N-(3,4,5-trimethoxyphenyl)indoline-5-sulfonamide

A solution of Example 2C (300 mg, 0.77 mmol) in THF (15 mL) at room temperature was treated with 1M LiAlH₄ (7.7 mL, 7.7 mmol) to form a solid which later dissolved to give a cloudy yellow solution, stirred overnight at room temperature, cooled to 0 °C, treated sequentially with water (0.3 mL), 15% NaOH (0.3 mL) and water (0.9 mL), stirred 30 minutes, and filtered to remove the aluminum complex. The layers comprising the filtrate were separated, and the organic layer was treated with ethyl acetate (50 mL), washed with water (2 x 20 mL), dried (Na₂SO₄), filtered and concentrated. The concentrate was purified by flash column chromatography on silica gel with 2% methanol:dichloromethane to provide 260 mg of the desired product.
MS (DCI/NH₃) *m*/*z* 379 (M+H)⁺;
¹H NMR (DMSO-d₆, 300 MHz) δ 9.76 (s, 1H), 7.45 (dd, J=2.1, 8.4 Hz, 1H), 7.34 (d, 1H), 6.46 (d, J=8.4 Hz, 1H), 6.37 (s, 2H), 3.65 (s, 6H), 3.55 (s, 3H), 3.42 (t, J=8.4 Hz, 2H) 2.92 (t, J=8.4 Hz, 2H) 2.76 (s, 3H).

### Example 6

### 1-methyl-5-nitro-N-(3,4,5-trimethoxyphenyl)-1H-indole-3-sulfonamide

### Example 6A

### 1-methyl-5-nitro-1H-indole-3-sulfonyl chloride

A solution of chlorosulfonic acid (1.9 mL, 28 mmol) and Na₂SO₄ (403 mg, 2.8 mmol) in dichloromethane (17 mL) was treated with a solution of 1-methyl-5-nitro-1*H*-indole (500 mg, 2.8 mmol) in dichloromethane (11 mL) over 1 hour, stirred for 30 minutes, and decanted to provide a thick brown oil. The oil was slowly treated with water (20 mL), stirred for 10 minutes, and filtered. The filtrate was dried in a vacuum oven to provide 80 mg of the desired compound which was used in the next step without further purification.

### Example 6B

### 1-methyl-5-nitro-N-(3,4,5-trimethoxyphenyl)-1H-indole-3-sulfonamide

3,4,5-trimethoxyaniline (47 mg) was processed as described in Example 1 (substituting Example 6A for 4-methoxybenzenesulfonyl chloride) to provide 59 mg of the desired product.
MS (DCI/NH₃) *m*/*z* 439 (M+NH₄)⁺;
¹H NMR (DMSO-d₆, 300 MHz) δ 10.20 (s, 1H), 8.71 (d, J=2.1 Hz, 1H), 8.34 (s, 1H), 8.15 (dd, J=2.1, 9 Hz, 1H), 7.76 (d, J=9 Hz, 1H), 6.40 (s, 2H), 3.91 (s, 3H), 3.61 (s, 6H) 3.51 (s, 3H).

### Example 7

### 5-amino-1-methyl-N-(3,4,5-trimethoxyphenyl)-1H-indole-3-sulfonamide

A solution of Example 6 (50 mg, 0.12 mmole) in 1:1 methanol:THF (2 mL) was treated with 10% palladium on carbon, stirred under hydrogen (1atm) for 2.5 hours, filtered through diatomaceous earth (Celite®), concentrated, redissolved in a small amount of methanol, treated with several drops of 1M HCl in diethyl ether until the solution became cloudy and acidic, treated with additional diethyl ether until a solid precipitated, filtered and dried in a vacuum oven to provide 35 mg of the desired the product.
MS (DCI/NH₃) *m*/*z* 392 (M+H)⁺ 409 (M+NH₄)⁺;
¹H NMR (DMSO-d₆, 300 MHz) δ 9.88 (s, 1H), 7.82 (s, 1H), 7.20 (d, J=9 Hz, 1H), 6.98 (d, J=2.4 Hz, 1H), 6.64 (dd, J=2.4, 9 Hz, 1H), 6.39 (s, 2H), 3.71 (s, 3H), 3.62 (s, 6H), 3.52 (s, 3H).

### Example 8

### 5-amino-N-(3,4,5-trimethoxyphenyl)-1H-indole-3-sulfonamide

Example 4B (100 mg) was processed as described in Example 7 to provide 80 mg of the desired product.
MS (DCI/NH₃) *mlz* 378 (M+H)⁺ 395 (M+NH₄)⁺;
¹H NMR (300 MHz, CDCl₃) δ 8.10 (d, 1H), 7.83 (m, 1H), 7.60 (d, 1H), 7.26 (m, 1H), 6.39 (s, 2H), 3.61 (s, 6H), 3.52 (s, 3H).

### Example 9

### N-(3,4,5-trimethoxyphenyl)-1H-indole-5-sulfonamide

A solution of Example 3 (0.909 g) and salcomine (0.082 g) in methanol (125 mL) was treated with O₂ gas over 18 hours and concentrated. The concentrate was purified by flash column chromatography on silica gel with 2% methanol/dichloromethane to provide the desired compound.
MS (DCI/NH₃) *m*/*z* 363 (M+H)⁺ 380 (M+NH₄)⁺;
¹H NMR (300 MHz, CDCl₃) δ 9.94 (s, 1H), 8.07 (s, 1H), 7.52 (m, 2H), 6.61 (m, 1H), 6.39 (s, 2H), 3.61 (s, 6H), 3.51 (s, 3H).

### Example 10

### 1-methyl-N-(3,4,5-trimethoxyphenyl)-1H-indole-5-sulfonamide

A solution of Example 9 (1.01 g, 2.8 mmol) in THF (100 mL) at 0 °C was treated with sodium bis(trimethylsilyl)amide (1M in THF, 7 mL, 6.89 mmol), stirred for 20 minutes, treated dropwise with CH₃I (195 µL, 3.1 mmol), stirred over 18 hours while warming to room temperature, treated with water, and extracted with ethyl acetate. The extract was dried (Na₂SO₄), filtered and concentrated. The concentrate was purified by flash column chromatography on silica gel with 1% methanol/dichloromethane to provide 684 mg of the desired compound.
MS (DCI/NH₃) *m*/*z* 377 (M+H)⁺ 394 (M+NH₄)⁺;
¹H NMR (DMSO-d₆, 300 MHz) δ 9.94 (s, 1H), 8.06 (t, 1H), 7.57 (m, 2H), 7.49 (d, J=3.3 Hz, 1H), 6.64 (d, J=3.3 Hz, 1H), 6.39 (s, 1H), 3.81 (s, 3H), 3.62 (s, 6H), 3.51 (s, 3H).

### Example 11

### N,1-dimethyl-N-(3,4,5-trimethoxyphenyl)-1H-indole-5-sulfonamide

A solution of example Example 9 (50 mg, 0.14 mmol) in THF at 0 °C was treated portionwise with NaH (60% dispersion in mineral oil, 26 mg, 0.70 mmol), stired for 20 minutes, treated dropwise with CH₃I (52 µL, 0.84 mmol), warmed to room temperature, stirred for 18 hours, treated with water, and extracted with ethyl acetate (10 mL). The organic layer was washed sequentially with water (5 mL) and brine (5 mL), dried (Na₂SO₄), filtered, and concentrated. The concentrate was purified by flash column chromatography on silica gel with 1% methanol:dichloromethane to provide 48 of the desired product.
MS (DCI/NH₃) *m*/*z* 391 (M+H)⁺;
¹H NMR (DMSO-d₆, 300 MHz) δ 7.86 (d, J=1.5 Hz, 1H), 7.64 (d, J=8.4 Hz, 1H), 7.54 (d, J=2.7 Hz, 1H), 7.32 (dd, J=1.5, 8.4 Hz, 1H), 6.65 (d, J=2.7 Hz, 1H), 6.29 (s, 2H), 3.86 (s, 3H), 3.64 (s, 3H), 3.58 (s, 6H), 3.06 (s, 3H).

### Example 12 (reference example)

### 3,4,5-trimethoxy-N-(4-methoxyphenyl)benenesulfonamide

### Example 12A

### 3,4,5-trimethoxybenzenesulfonyl chloride

The procedure in *J. Het. Chem.* **23**,1253 (1986) was followed. A solution of 3,4,5-trimethoxyaniline (5.0 g) in acetic acid (26 mL) and 12M HCl (47 mL) at -10-5 °C was treated slowly with a solution of NaNO₂ (2 g) in water (7 mL), stirred at -5 °C for another 30 minutes, added in portions to a cold (-5 °C) solution of CuCl₂ and SO₂ in acetic acid (35 mL) and water (6 mL), stirred at -5-0 °C for 3 hours, warmed to room temperature overnight, poured over ice, filtered, and dried to provide the desired product.

### Example 12B

### 3,4,5-trimethoxy-N-(4-methoxyphenyl)benenesulfonamide

A solution of 4-methoxyaniline (139 mg, 1.1 mmol) in pyridine (2 mL) was treated with Example 12A (300 mg, 1.1 mmol) in THF (2 mL), stirred at room temperature for 18 hours, concentrated, redissolved in THF (1 mL), treated with water with stirring, and filtered to provide 300 mg of the desired product.
MS (DCI/NH₃) *m*/*z* 353 (M+H)⁺ and 371 (M+NH₄)⁺;
¹H NMR (300 MHz, CDCl₃) δ 6.99 (d, J=9 Hz, 1H), 6.80 (d, J=9 Hz, 1H), 6.85 (s, 2H), 3.87 (s, 3H), 3.77 (s, 3H), 3.76 (s, 6H).

### Example 13 (reference example)

### N-(1-methyl-1H-indol-5-yl)-3,4,5-trimethoxybenzenesulfonamide

Example 12A (456 mg) was processed as described in Example 12B (substituting 1*H*-indol-5-amine for 4-methoxyaniline) to provide 480 mg of the desired product.
MS (DCI/NH₃) *m*/*z* 377 (M+H)⁺ and 394 (M+NH₄)⁺;
¹H NMR (300 MHz, CDCl₃) δ 7.32 (d, J=3 Hz, 1H), 7.19 (d, J=8.4 Hz, 1H), 7.05 (d, J=3 Hz, 1H), 6.90 (dd, J=3.0, 8.4 Hz, 1H), 6.86 (s, 2H), 6.40 (d, J=3 Hz, 1H), 3.85 (s, 3H), 3.76 (s, 3H), 3.68 (s, 6H).

### Example 14 (reference example)

### 3,4,5-trimethoxy-N-(2-(dimethylamino)ethyl)-N-(1-methyl-1H-indol-5-yl)benzenesulfonamide

A solution of Example 13 (50 mg, 0.13 mmol) in THF (2 mL) was treated sequentially with triphenylphosphine (52 mh, 0.19 mmol), N,N-dimethyl-2-aminoethanol (17 µL, 0.16 mmol), and diethylazodicarboxylate (31 µL, 0.19 mmol), stirred at room temperature overnight, treated with silica gel, and concentrated. The mixture was purified by flash column chromatography on silica gel with 1% methanol:dichloromethane to provide 46 mg of the desired product
MS (DCI/NH₃) *m*/*z* 448 (M+H)⁺ and 470 (M+Na)⁺;
¹H NMR (300 MHz, CDCl₃) δ 7.40 (d, J=8.7 Hz, 1H), 7.38 (d, J=3 Hz, 1H), 7.26 (d, J=2.1 Hz, 1H), 6.83 (dd, J=2.1, 8.7 Hz, 1H), 6.79 (s, 2H), 6.41 (d, J=3 Hz, 1H), 3.78 (s, 3H), 3.75 (s, 3H), 3.70 (s, 6H), 3.64 (t, 2H), 2.22 (t, 2H) 2.08 (s, 6H).

### Example 15

### 1H-indol-5-ol,(3,4,5-trimethoxybenzenesulfonate) ester

A solution of 5-hydroxyindole (CAS number 13523-92-7,253 mg, 1.9 mmol) in dichloromethane (15 mL) and pyridine (0. 5 mL) was treated sequentially with Example 12A (507 mg, 1.9 mmol) and a catalytic amount of DMAP, stirred for 1 week, and washed with saturated CuSO₄. The organic layer was dried (MgSO₄), filtered, and concentrated. Chromatography ofthe concentrate on silica gel with 30% ethyl acetate/hexane provided 520 mg of the desired compound as a white crystalline solid.
MS(ESI/NH₃) *m*/*z* 364(M+H)⁺;
¹H NMR (300 MHz, CDCl₃) δ 8.24 (br s, 1H), 7.29-7.25 (m, 3H), 7.02 (s, 2H), 6.82 (dd, J=2.2, 8.8, 1H), 6.51 (m, 1H), 3.92 (s, 3H), 3.78 (s, 6H).

### Example 16

### 1-methyl-1H-indol-5-yl 3,4,5-trimethoxybenzenesulfonate

Example 12A (726 mg) was processed as described in Example 15 (substituting 1-methyl-1*H* indol-5-ol for 5-hydroxyindole to provide 650 mg of the desired product.
MS (ESI/NH₃) *m*/*z* 395 (M+NH₄)⁺;
¹H NMR (300 MHz, CDCl₃) δ 7.24 (d, J=2.4, 1H), 7.19 (d, J=8.8, 1H), 7.09 (d, J=3.1, 1H), 7.02 (s, 2H), 6.85 (dd, J=2.4, 8.8, 1H), 6.43 (dd, J=0.7,3.1, 1H), 3.92 (s, 3H), 3.79 (s, 6H), 3.77 (s, 3H).

### Example 17

### 1-ethyl-N-(3,4,5-trimethoxyphenyl)-1H-indole-5-sulfonamide

Example 9 (50 mg) was processed as described in Example 10 (substituting ethyl iodide for methyl iodide) to provide 40 mg of the desired product.
MS (DCI/NH₃) m/z 391 (M+H)⁺ and 408 (M+NH₄)⁺;
¹H NMR (300 MHz, DMSO-d₆) δ 9.93 (s, 1H), 8.06 (d, J=2.1 Hz, 1H), 7.70 (d, J=3 Hz, 1H), 7.63 (m, 1H), 7.60 (d, J=2.1 Hz, 1H), 6.62 (d, J=3Hz, 1H), 6.39 (s, 2H), 4.23 (q, J=7.5 Hz, 2H), 3.61 (s, 6H), 3.52 (s, 3H), 1.34 (t, J=7.5 Hz, 3H).

### Example 18

### 3,4,5-trimethoxy-N-(6-quinolinyl)benzenesulfonamide

Example 12A (185 mg) was processed as described in Example 12B (substituting 6-aminoquinoline for 4-methoxyaniline) to provide 180 mg of the desired product.
MS (DCI/NH₃) *m*/*z* 375 (M+H)⁺;
¹H NMR (300 MHz, DMSO-d₆) δ 10.58 (s, 1H), 8.77 (dd, J=1.5 Hz, 4.0 Hz, 1H), 8.31 (d, J=8.7 Hz 1H), 7.91 (d, J=9 Hz, 1H), 7.68 (d, J=2.4, 1H), 7.53 (dd, J=9 Hz, 2.4 Hz, 1H), 7.46 (dd, J=8.7 Hz, 4.2 Hz, 1H), 3.73 (s, 6H), 3.65 (s, 3H).

### Example 19

### N-(2-hydroxyethyl)-1-methyl-N-(3,4,5-trimethoxyphenyl)-1H-indole-5-sulfonamide

Example 10 (50 mg) was processed as described in Example 14 (substituting ethylene glycol for *N*,*N*-dimethyl-2-aminoethanol) to provide 25.5 mg of the desired product.
MS (DCI/NH₃) *m*/*z* 421 (M+H)⁺;
¹H NMR (300 MHz, DMSO-d₆) δ 7.86 (d, J=1.8 Hz, 1H), 7.64 (d, J=8.7 Hz, 1H), 7.54 (d, J=3.3 Hz, 1H), 7.32 (dd, J=8.7 Hz, 1.8 Hz, 1H), 6.65 (d, J=3.3 Hz, 1H), 6.29 (s, 2H), 4.03 (q, J=6.9 Hz, 2H), 3.84 (s, 3H), 3.64 (s, 3H), 3.58 (s, 6H), 2.52 (d, J=6.9 Hz, 2H).

### Example 20

### N-(2-fluoroethyl)-1-methyl-N-(3,4,5-trimethoxyphenyl)-1H-indole-5-sulfonamide

Example 10 (50 mg) was processed as described in Example 14 (substituting 2-fluoroethanol for *N*,*N*-dimethyl-2-aminoethanol) to provide 30 mg of the desired product.
MS (DCI/NH₃) *m*/*z* 423 (M+H)⁺;
¹H NMR (300 MHz, DMSO-d₆) δ 7.90 (d, J=1.8 Hz, 1H), 7.64 (d, J=8.7 Hz, 1H), 7.54 (d, J=3.3 Hz, 1H), 7.40 (dd, J=8.7 Hz, 1.8 Hz, 1H), 6.63 (d, J=3.3 Hz, 1H), 6.23 (s, 2H), 4.48 (t, 2H), 4.32 (t, 2H), 3.86 (s, 3H), 3.65 (s, 3H), 3.54 (s, 6H).

### Example 21

### N-ethyl-1-methyl-N-(3,4,5-trimethoxyphenyl)-1H-indole-5-sulfonamide

Example 10 (50 mg) was processed as described in Example 14 (substituting ethanol for *N,N*-dimethyl-2-aminoethanol) to provide 35 mg of the desired product.
MS (DCI/NH₃) *m*/*z* 405 (M+H)⁺;
¹H NMR (300 MHz, DMSO-d₆) δ 7.89 (d, J=1.8 Hz, 1H), 7.64 (d, J=8.7 Hz, 1H), 7.54 (d, J=3.3 Hz, 1H), 7.40 (dd, J=8.7 Hz, 1.8 Hz, 1H), 6.64 (d, J=3.3 Hz, 1H), 6.22 (s, 2H), 3.86 (s, 3H), 3.65 (s, 3H), 3.56 (s, 6H), 3.49 (q, 2H), 0.97 (t, 3H).

### Example 22

### N-((dimethylamino)acetyl)-1-methyl-N-(3,4,5-trimethoxyphenyl)-1H-indole-5-sulfonamide

A solution of Example 10 (1.00 g, 2.66 mmol) in dichloromethane (25 mL) at room temperature was treated with DCC (2.75 g, 13.3 mmol), 4-pyrrolidinylpyridine (0.20 g, 1.32 mmol) and *N,N*-dimethylglycine (0.68 g, 6.40 mmol), stirred for 16 hours, diluted with dichloromethane, and filtered. The filtrate was washed with water, dried (MgSO₄), filtered, and concentrated. The concentrate was treated with dichloromethane, filtered, and concentrated. The concentrate was purified by flash column chromatography on silica gel with 1.5% methanol/dichloromethane, dissolved in dichloromethane (5 mL) and diethyl ether (5 mL), treated with 4M HCl in dioxane (0.55 mL), stirred for 10 minutes, treated with ether, and filtered to provide the desired product.
mp: 200-203 °C;
MS (ESI(+)) *m*/*z* 462 (M+H)⁺;
¹H NMR (DMSO-d₆): δ 8.36 (d,*J*=1.8 Hz, 1H), 7.78 (dd, *J*₁=8.7 Hz, *J*₂=1,8 Hz, 1H), 7.73 (d, J=8.7 Hz, 1H), 7.61 (d, *J*=3.0 Hz, 1H), 6.76 (d,*J*=3.0 Hz, 1H), 6.73 (s, 2H), 3.92 (s, 2H), 3.82 (s, 3H), 3.82 (s, 6H), 3.75 (s, 3H), 2.67 (s, 6H);
Anal. calcd. for C₂₂H₂₇N₃O₆S·HCl·1.5H₂O: C, 50.43; H, 5.77; N, 8.02. Found: C, 50.50; H, 5.93; N, 8.01.

### Example 23

### 1-methyl-N-(((2S)-1-methylpyrrolidinyl)carbonyl)-N-(3,4,5-trimethoxyphenyl)-1H-indole-5-sulfonamide

The desired product was prepared by substituting *N*-methyl-L-proline for *N,N*-dimethylglycine in Example 22.
MS (APCI(+)) *m*/*z* 488 (M+H)⁺;
¹H NMR (DMSO-d₆) δ 8.36 (d, J=2 Hz, 1H), 7.75 (m, 2H), 7.61 (d, *J*=3 Hz, 1H), 6.82 (s, 2H), 4.76 (d, *J=*3 Hz, I H), 4.06 (m, 1H), 3.90 (s, 3H), 3.81 (s, 6H), 3.77 (s, 3H), 3.00 (m, 2H), 2.69 (s, 3H), 1.90 (m, 3H), 1.73 (m, 1H).

### Example 24

### N-((2S)-2-(dimethylamino)-3-methylbutanoyl)-1-methyl-N-(3,4,5-trimethoxylphenyl)-1H-indole-5-sulfonamide

The desired product was prepared by substituting *N*,*N*-dimethyl-L-valine for *N,N*-dimethylglycine in Example 22.
MS (ESI(+)) *m*/*z* 504 (M+H)⁺;
¹H NMR (DMSO-d₆) δ 8.38 (s, 1H), 7.75 (m, 2H), 7.60 (d, *J=* 3Hz, 1H), 6.76 (d, *J=*3 Hz, 1H), 6.55 (br s, 2H), 3.89 (s, 3H), 3.82 (m, 1H), 3.79 (s, 9H), 2.68 (m, 3H), 2.55 (br s, 3H), 2.26 (br s, 1H), 0.91 (m, 3H), 0.75 (m, 3H).

### Example 25

### N-((2S)-2-amino-3-methylbutanoyl)-1-methyl-N-(3,4,5-trimethoxyphenyl)-1H-indole-5-sulfonamide

A solution of Example 10 (400 mg, 1.06 mmol) in dichloromethane (8 mL) at room temperature was treated with DCC (482 mg, 2.42 mmol), 4-pyrrolidinopyridine (16 mg, 0.11 mmol), and *N*-(*tert*-butoxycarbonyl)-L-valine (462 mg, 2.13 mmol), stirred for 16 hours, treated with additional DCC (50 mg, 0.25 mmol) and *N*-(*tert*-butoxycarbonyl)-L-valine (50 mg, 0.23 mmol), stirred for 4 hours, and filtered. The filtrate was treated with dichloromethane (50 mL), washed with water, dried (Na₂SO₄), filtered, and concentrated. The concentrate was purified by flash column chromatography on silica gel with 2% methanol/dichloromethane, dissolved in dioxane (5 mL), treated with 4M HCl in dioxane, stirred for 2 hours, treated with ether (75 mL), filtered, and dried to provide the desired product.
MS (ESI(+)) *m*/*z* 576 (M+H)⁺;
¹H NMR (DMSO-d₆) δ 8.33 (m, 1H), 7.73 (m, 2H), 7.60 (d, *J*=3 Hz, 1H), 6.75 (d, *J*=3 Hz, 1H), 6.65 (br s, 2H), 3.80 (br s, 7H), 3.76 (s, 3H), 3.57 (s, 3H), 2.10 (m, 1H), 0.80 (d, *J=*2.4 Hz, 3H), 0.78 (d, *J*=2.7 Hz, 3H).

### Example 26

### 1-methyl-N-((2S)-2-(methylamino)propanoyl)-N-(3,4,5-trimethoxyphenyl)-1H-indole-5-sulfonamide

The desired product was prepared by substituting *N*-(*tert*-butoxycarbonyl)-L-*N*-methylalanine for *N*-(*tert*-butoxycarbonyl)-L-valine in Example 25.
MS (DCI/NH₃) *m*/*z* 462 (M+H)⁺;
¹H NMR (DMSO-d₆) δ 8.95 (br s, 2H), 8.33 (m, 1H), 7.68-7.78 (m, 2H), 7.60 (d, *J*=4 Hz, 1H), 6.72 (d, *J*=4 Hz, 1H), 6.70 (br s, 2H), 3.89 (s, 3H), 3.81 (s, 6H), 3.77 (s, 3H), 3.62-3.71 (m, 1H), 2.37 (s, 3H), 1.38 (d, *J*=8 Hz, 3H).

### Example 27

### =N-((2S)-2-amino-2-phenylethanoyl)-1-methyl-N-(3,4,5-trimethoxyphenyl)-1H-indole-5-sulfonamide

The desired product was prepared by substituting (*S*)-*N*-(*tert*-butoxycarbonyl)-2-phenylglycine for *N*-(*tert*-butoxycarbonyl)-L-valine in Example 25.
MS (ESI(+)) *m*/*z* 510 (M+H)⁺; 532 (M+Na)⁺;
¹H NMR (DMSO-d₆) δ 8.52 (br s, 3H), 8.34 (m, 1H), 7.74 (s, 1H), 7.63(d, *J*= 3.4 Hz, 1H), 7.41-7.27 (m, 3 H), 6.83-6.67 (m, 4H), 5.51 (br s, 1H), 4.92 (s, 1H), 3.91 (s, 3H), 3.87-3.85 (m, 3H), 3.73 (s, 3H), 3.57 (s, 3H).

### Example 28

### N-((2S)-2-amino-3-phenylpropanoyl)-1-methyl-N-(3,4,5-trimethoxyphenyl)-1H-indole-5-sulfonamide

The desired product was prepared by substituting *N*-(*tert*-butoxycarbonyl)-L-phenylalanine for *N*-(*tert-*butoxycarbonyl)-L-valine in Example 25.
MS (ESI(+)) *m*/*z* 524 (M+H)⁺; 546 (M+Na)⁺;
¹H NMR (DMSO-d₆) δ 8.44 (br s, 3H), 8.32 (m, 1H), 7.74 (d, *J* = 1.6 Hz, 1H), 7.63 (d, *J*=3.4 Hz, 1H), 7.28-7.11 (m, 3H), 6.82-6.77 (m, 4H), 6.00 (br s, 1H), 3.91 (s, 3H), 3.72-3.67 (m, 9H).

### Example 29

### 1-methyl-N-((2S)-pyrrolidinylcarbonyl)-N-(3,4,5-trimethoxyphenyl)-1H-indole-5-sulfonamide

The desired product was prepared by substituting *N*-(*tert*-butoxycarbonyl)-L-proline for *N*-(*tert*-butoxycarbonyl)-L-valine in Example 25.
MS (ESI(+)) *m*/*z* 474 (M+H)⁺;
¹H NMR (DMSO-d₆) δ 8.35 (m, 1H), 7.76 (m, 2H), 7.61 (d, *J=3* Hz, 1H), 6.75 (d, *J=3* Hz, 1H), 6.70 (br s, 2H), 4.03 (m, 1H), 3.89 (s, 3H), 3.81 (br s, 6H), 3.76 (s, 3H), 3.20 (m, 2H), 1.95-1.75 (m, 4H).

### Example 30

### N-((2S)2,6-diaminohexanoyl)-1-methyl-N-(3,4,5-trimethoxyphenyl)-1H-indole-5-sulfonamide

The desired product was prepared by substituting *N,N*-di-(*tert*-butoxycarbonyl)-L-lysine for *N*-(*tert*-butoxycarbonyl)-L-valine in Example 25.
MS (ESI(+)) *m*/*z* 505 (M+H)⁺;
¹H NMR (DMSO-d₆) δ 8.30 (m, 1H), 7.73 (m, 2H), 7.60 (d, *J=3* Hz, 1H), 6.75 (d, *J=3* Hz, 1H), 6.65 (br s, 2H), 3.89 (s, 3H), 3.80 (m, 6H), 3.76 (s, 3H), 3.59 (m, 1H), 2.67 (m, 2H), 1.75-1.20 (m, 6H).

### Example 31

### N-((2S)-2-amino-3-(1H-imidazol-5-yl)propanoyl)-1-methyl-N-3,4,5-trimethoxyphenyl)-1H-indole-5-sulfonamide

The desired product was prepared by substituting *N*-(*tert*-butoxycarbonyl)-L-histidine for *N*-(*tert*-butoxycarbonyl)-L-valine in Example 25.
mp: 175-177 °C;
MS (ESI(+)) *m*/*z* 514 (M+H)⁺;
¹H NMR (DMSO-d₆) δ 9.03 (s, 1H), 8.29 (s, 1H), 7.71 (m, 2H), 7.61 (d, *J=3* Hz, 1H), 7.28 (s, 1H), 6.75 (d, *J*=3 Hz, 1H), 6.70 (br s, 2H), 4.01 (m, 1H), 3.89 (s, 3H), 3.79 (br s, 6H), 3.76 (s, 3H), 1.72 (m, 2H).

### Example 32

### (2S)-2-amino-4-oxo-4-(3,4,5-trimethoxy((1-methyl-1H-indol-5-yl)sulfonyl)anilino)butanoic acid

The desired product was prepared by substituting *N*-(*tert*-butoxycarbonyl)-L-aspartic acid 1-*tert*-butyl ester for *N*-(*tert*-butoxycarbonyl)-L-valine in Example 25.
mp: 156-159 °C;
MS (ESI(+))*m*/*z* 492(M+H)⁺;
¹H NMR (DMSO-d₆) δ 8.35 (d, *J=2* Hz), 7.75 (m, 2H), 7.60 (d, *J*=1H), 6.73 (d, *J*=3 Hz, 1 H), 6.63 (br s, 2H), 4.01 (m, 1H), 3.88 (s, 3H), 3.82 (s, 6H), 3.76 (s, 3 H), 2.80-2.70 (m, 2H).

### Example 33

### (3S)-3-amino-4-oxo-4-(3,4,5-trimethoxy((1-methyl-1H-indol-5-yl)sulfonyl)anilino)butanoic acid

The desired product was prepared by substituting *N*-(*tert*-butoxycarbonyl)-L-aspartic acid 4*-tert-*butyl ester for *N*-(*tert*-butoxycarbonyl)-L-valine in Example 25.
MS (ESI(+)) *m*/*z* 492 (M+H)⁺;
¹H NMR (DMSO-d₆): δ 8.33 (m, 1H), 7.73 (m, 2H), 7.61 (d, *J=3* Hz, 1H), 6.62 (d, *J=*3 Hz, 1H), 6.40 (s, 2H), 3.95 (m, 1H), 3.88 (s, 3H), 3.76 (s, 6H), 3.74 (s, 3H), 2.75 (m, 2H).

### Example 34

### (2S)-2-amino-5-oxo-5-(3,4,5-trimethoxy-((1-methyl-1H-indol-5-yl)sulfonyl)anilino)pentanoic acid

The desired product was prepared by substituting *N*-(*tert*-butoxycarbonyl)-L-glumatic acid 1-*tert*-butyl ester for *N*-(*tert*-butoxycarbonyl)-L-valine in Example 25.
MS (ESI(+)) *m*/*z* 506 (M+H)⁺;
¹H NMR (DMSO-d₆) δ 8.31 (br s, 1H), 7.75 (dd, *J*₁=9 Hz, *J*₂=2 Hz, 1H), 7.69 (d, *J*=9Hz, 1H), 7.58 (d, *J*=3 Hz, 1H), 6.72 (d, *J*=3 Hz, 1H), 6.69 (s, 1H), 6.66 (s, 1H), 4.06 (m, 1H), 3.88 (s, 3H), 3.80 (s, 6H), 3.72 (s, 3H), 2.30 (m, 1H), 2.22 (m, 1H), 1.95 (m, 1H), 1.80 (m, 1H).

### Example 35

### (4S)-4-amino-5-oxo-5-(3,4,5-trimethoxy((1-methyl-1H-indol-5-yl)sulfonyl)anilino)pentanoic acid

The desired product was prepared by substituting *N*-(*tert*-butoxycarbonyl)-L-glumatic acid 5-*tert*-butyl ester for *N*-(*tert*-butoxycarbonyl)-L-valine in Example 24.
MS (ESI(+)) *m*/*z* 506 (M+H)⁺;
¹H NMR (DMSO-d₆) δ 8.33 (m, 1H), 7.74 (m, 2H), 7.60 (d, *J=3* Hz, 1H), 6.75 (d, *J=3* Hz, 1H), 6.71 (br s, 2H), 4.06 (m, 1H), 3.89 (s, 3H), 3.80 (s, 6H), 3.75 (s, 3H), 3.72 (s, 3H), 2.19 (m, 2H), 1.95-1.70 (m, 2H).

### Example 36

### N-((bis(2-methoxyethyl)amino)acetyl)-1-methyl-N-(3,4,5-trimethoxyphenyl)-1H-indole-5-sulfonamide

### Example 36A

### benzyl (bis(2-methoxyethyl)amino)acetate

A solution of benzyl bromoacetate (28.9 g; 96.7 mmol) in dichloromethane (100 mL) at room temperature was treated with 2-methoxy-*N*-(2-methoxyethyl)ethanamine (40.6 g; 305 mmol), stirred for 30 minutes, diluted with dichloromethane, washed sequentially with saturated NH₄Cl, water, and brine, dried (Na₂SO₄), filtered, and concentrated. The concentrate was purified by flash column chromatography on silica gel with 40% diethyl ether/dichloromethane to provide the desired product.

### Example 36B

### (bis(2-methoxyethyl)amino)acetic acid

A solution of Example 36A (19.4 g; 69 mmol) and 10% Pd/C (3.5 g) in methanol (150 mL) at room temperature was stirred under 4 atm of H₂ for 17 hours, filtered through diatomaceous earth (Celite®), and concentrated to provide the desired product.

### Example 36C

### N-((bis(2-methoxyethyl)amino)acetyl)-1-methyl-N-(3,4,5-trimethoxyphenyl)-1H-indole-5-sulfonamide

The desired product was prepared by substituting Example 36B for *N,N*-dimethylglycine in Example 22.
MS (DCI/NH₃) *m*/*z* 550 (M+H)⁺;
¹H NMR (DMSO-d₆) δ 8.29 (d, *J*=2Hz, 1H), 7.77-7.68 (m, 2H), 7.57 (d, *J*=4 Hz, 1H), 6.73 (d, *J*=4 Hz, 1H), 6.61 (s, 2H), 3.88 (s, 3H), 3.78 (s, 6H), 3.72 (s, 3H), 3.19 (s, 2H), 3.16 (t, *J*=6 Hz, 4H), 3.04 (s, 6H), 2.61 (t, *J*=6 Hz, 4H);
Anal. calcd. for C₂₆H₃₆ClN₃O₈S: C, 53.28; H, 6.19; N, 7.17; Cl, 6.05. Found: C, 53.28; H, 6.03; N, 7.10; Cl, 5.97.

### Example 37

### 1-methyl-N-(4-morpholinylacetyl)-N-(3,4,5-trimethoxyphenyl)-1H-indole-5-sulfonamide

The desired product was prepared by substituting morpholine for 2-methoxy-*N*-(2-methoxyethyl)ethanamine in Example 36.
MS (DCI/NH₃) *m*/*z* 504 (M+H)⁺;
¹H NMR (DMSO-d₆) δ 8.29-7.77 (d, *J*=2 Hz, 1H), 7.68 (m, 2H), 7.57 (d, *J*=4 Hz, 1H), 6.73 (d, *J*=4 Hz, 1H), 6.63 (s, 2H), 3.88 (s, 3H), 3.80 (s, 6H), 3.72 (s, 3H), 3.47-3.41 (m, 4H), 2.94 (s, 2H), 2.31-2.27 (m, 4H).

### Example 38

### 1-methyl-N-((4-methyl-1-piperazinyl)acetyl)-N-(3,4,5-trimethoxyphenyl-1H-indole-5-sulfonamide

The desired product was prepared by substituting *N*-methylpiperazine for 2-methoxy-*N*-(2-methoxyethyl)ethanamine in Example 36.
MS (DCI/NH₃) *m*/*z* 517 (M+H)⁺;
¹H NMR (DMSO-d₆) δ 8.29 (d, *J*=2 Hz, 1H), 7.77-7.68 (m, 2H), 7.57 (d, *J*=4 Hz, 1H), 6.73 (d, *J*=4 Hz, 1H), 6.61 (s, 2H), 3.88 (s, 3H), 3.78 (s, 6H), 3.72 (s, 3H), 2.91 (s, 2H), 2.29-2.13 (m, 8H), 2.07 (s, 3H).

### Example 39

### N-(4-(aminomethyl)benzoyl)-1-methyl-N-(3,4,5-trimethoxyphenyl)-1H-indole-5-sulfonamide

### Example 39A

### 4-(((tert-butoxycarbonyl)amino)methyl)benzoic acid

A solution of 4-(aminomethyl)benzoic acid (1.51 g, 10 mmol), di(*tert*-butyl) dicarbonate (2.62 g, 12 mmol), and sodium hydroxide (0.48 g, 12 mmol) in tert-butanol (20 mL) was stirred for 16 hours, treated with water (200 mL), and extracted with hexanes. The aqueous layer was cooled to 5 °C, adjusted to pH 4 with 1M NaHSO₄, and extracted with ethyl acetate. The combined extracts were dried (Na₂SO₄), filtered, and concentrated to provide the desired product.
MS (ESI(+)) *m*/*z* 252 (M+H)⁺, 269 (M+NH₄)⁺;
¹H NMR (DMSO-d₆) δ 8.06 (d,*J* = 8.5 Hz, 2H), 7.38 (d, *J* = 8.5 Hz, 2H), 4.95 (br s, 1H), 4.40 (d, *J* = 5.9 Hz, 2 H), 1.48 (s, 9H).

### Example 39B

### N-(4-(aminomethyl)benzoyl)-1-methyl-N-(3,4,5-trimethoxyphenyl)-1H-indole-5-sulfonamide

The desired product was prepared by substituting Example 39A for *N-(tert*-butoxycarbonyl)-L-valine in Example 25.
MS (ESI(+)) *m*/*z* 510 (M+H)⁺;
¹H NMR (DMSO-d₆) δ 8.35 (br s, 3H), 8.24 (m, 1H), 7.74 (s, 1H), 7.68(m, 2H), 7.59 (m, 3 H), 7.34 (m, 2H), 6.73 (m, 1H), 6.57 (s, 2H), 3.95 (m, 2H), 3.88 (s, 3H), 3.64 (m, 6H), 3.56 (s, 3H).

Following Scheme 1 and the examples described above, the following compounds can be prepared:

### Example 40

### N-(2-aminoacetyl)-1-methyl-N-(3,4,5-trimethoxyphenyl)-1H-indole-5-sulfonamide

### Example 41

### N-((2S)-2-aminopropanoyl)-1-methyl-N-(3,4,5-trimethoxyphenyl)-1H-indole-5-sulfonamide

### Example 42

### N-(3-aminopropanoyl)-1-methyl-N-(3,4,5-trimethoxyphenyl)-1H-indole-5-sulfonamide

### Example 43

### (2S)-2-amino-N-((1S)-1-methyl-2-oxo-2-(3,4,5-trimethoxy((1-methyl-1H-indol-5-yl)sulfonyl)anilino)ethyl)propanamide

### Example 44

### N-((2S)-2-amino-3-hydroxypropanol)-1-methyl-N-(3,4,5)-trimethoxyphenyl)-1H-indole-5-sulfonamide

## Claims

1. A compound having formula (I) 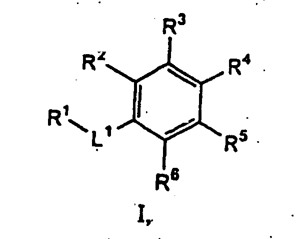 or a pharmaceutically acceptable salt thereof, wherein L¹ is selected from the group consisting of
(1) -OS(O)₂-, and
(2) -SO₂NR⁷-, wherein R⁷ is selected from the group consisting of
(a) hydrogen,
(b) alkanoyl,
(c) alkyl,
(d)aryloyl, wherein the aryl is phenyl,
wherein (b)-(d) can be optionally substituted with one or two substituents independently selected from the group consisting of
(i) hydroxyl,
(ii) halo,
(iii) carboxy,
(iv) phenyl,
(v) imidazolyl,
(vi) heterocycloalkyl selected from the group consisting of morpholinyl and piperazinyl,
(vii) -NR^{c}R^{d}, wherein R^{c} and R^{d} are independently selected from the group consisting of
(1') hydrogen,
(2') alkyl,
and
(3') alkoxyalkyl,
and
(viii) -(alkylene)-NR^{c}R^{d},
wherein for (v) and (vi), the imidazolyl and the heterocycloalkyl can be optionally substituted with one alkyl substituent,
and
(e) pyrrolidinylcarbonyl, wherein the pyrrolidinylcarbonyl can be optionally substituted with one alkyl substituent,
wherein (1)-(2) are shown with their left ends attached to R¹ and their right ends attached to the phenyl ring;
R¹ is heteroaryl, wherein the heteroaryl is selected from the group consisting of indolinyl and indolyl, and wherein the heteroaryl can be optionally substituted with one or two substituents independently selected from the group consisting of
(a) carboxaldehyde,
(b) nitro,
(c) alkyl, and
(d)-NH₂;
R² and R⁶ are hydrogen;
and
R³, R⁴, and R⁵ are alkoxy.

2. A compound according to Claim 1, wherein L¹ is -SO₂NR⁷-.

3. A compound according to Claim 2, wherein R³, R⁴, and R⁵ are methoxy.

4. A compound according to Claim 3, wherein R¹ is optionally substituted 2,3-dihydro-1*H*-indol-5-yl.

5. A compound according to Claim 3 selected from the group consisting of
1-formyl-*N*-(3,4,5-trimethoxyphenyl)indoline-5-sulfonamide,
*N*-(3,4,5-trimethoxyphenyl)indoline-5-sulfonamide, and
1-methyl-*N*-(3,4,5-trimethoxyphenyl)indoline-5-sulfonamide.

6. A compound according to Claim 3, wherein R¹ is optionally substituted 1*H*-indol-3-yl.

7. A compound according to Claim 6 selected from the group consisting of
5-nitro-*N*-(3,4,5-trimethoxyphenyl)-1*H*-indole-3-sulfonamide,
1-methyl-5-nitro-*N*-(3,4,5-trimethoxyphenyl)-1*H*-indole-3-sulfonamide,
5-amino-1-methyl-*N*-(3,4,5-trimethoxyphenyl)-1*H*-indole-3-sulfonamide, and
5-amino-*N*-(3,4,5-trimethoxyphenyl)-1*H*-indole-3-sulfonamide.

8. A compound according to Claim 3 wherein R¹ is optionally substituted 1*H*-indol-5-yl.

9. A compound according to Claim 8 selected from the group consisting of
*N*-(3,4,5-trimethoxyphenyl)-1*H*-indole-5-sulfonamide,
1-methyl-*N*-(3,4,5 trimethoxyphenyl)-1*H*-indole-5-sulfonamide,
*N*,1-dimethyl-N-(3,4,5-trimethoxyphenyl)-1*H*-indole-5-sulfonamide,
1-ethyl-*N*-(3,4,5-trimethoxyphenyl)-1*H*-indole-5-sulfonamide,
*N*-(2-hydroxyethyl)-1-methyl-*N*-(3,4,5-trimethoxyphenyl)-1*H*-indole-5-sulfonamide,
*N*-(2-fluoroethyl)-1-methyl)-*N*-(3,4,5-trimethoxyphenyl)-1*H*-indole-5-sulfonamide,
*N*-ethyl-1-methyl-*N*-(3,4,5-trimethoxyphenyl)-1*H*-indole-5-sulfonamide,
*N*-((dimethylamino)acetyl)-1-methyl-*N*-(3,4,5-trimethoxyphenyl)-1*H*-indole-5-sulfonamide,
1-methyl-*N*-(((2*S*)-1-methylpyrrolidinyl)carbonyl)-*N*-(3,4,5-trimethoxyphenyl)-1*H*-indole-5-sulfonamide,
*N*-((2*S*)-2-(dimethylamino)3-methylbutanoyl)-1-methyl-*N*-(3,4,5-trimethoxyphenyl)-1*H*-indole-5-sulfonamide,
*N*-((2*S*)-2-amino-3-methylbutanoyl)-1-methyl-*N*-(3,4,5-trimethoxyphenyl)-1*H*-indole-5-sulfonamide,
1-methyl-*N*-((2*S*)-2-methylamino)propanoyl)-*N*-(3,4,5-trimethoxyphenyl)-1*H*-indole-5-sulfonamide,
*N*-((2*S*)-2-amino-2-phenylethanoyl)-1-methyl-*N*-(3,4,5-trimethoxyphenyl)-1*H* indole-5-sulfonamide,
*N*-((2*S*)-2-amino-3-phenylpropanoyl)-1-methyl-*N*-(3,4,5-trimethoxyphenyl)-1*H*-indole-5-sulfonamide,
1-methyl-*N*-((2*S*)-pyrrolidinylcarbonyl)-*N*-(3,4,5-trimethoxyphenyl)-1*H*-indole-5-sulfonamide,
*N*-((2*S*)-2,6-diaminohexanoyl)-1-methyl-*N*-(3,4,5-trimethoxyphenyl)-1*H*-indole-5-sulfonamide,
*N*-((2*S*)-2-amino-3-(1*H*-imidazol-5-yl)propanoyl)-1-methyl-*N*-(3,4,5-trimethoxyphenyl)-1*H*-indole-5-sulfonamide,
(2*S*)-2-amino-4-oxo-4-(3,4,5-trimethoxy((1-methyl-1*H*-indol-5-yl)sulfonyl)anilino)butanoic acid,
(3*S*)-3-amino-4-oxo-4-(3,4,5-trimethoxy((1-methyl-1*H*-indol-5-yl)sulfonyl)anilino)butanoic acid,
(2*S*)-2-amino-5-oxo-5-(3,4,5-trimethoxy((1-methyl-1*H*-indol-5-yl)sulfonyl)anilino)pentanoic acid,
(4*S*)-4-amino-5-oxo-5-(3,4,5-trimethoxy((1-methyl-1*H*-indol-5-yl)sulfonyl)anilino)pentanoic acid,
*N*-((bis(2-methoxyethyl)amino)acetyl)-1-methyl-*N*-(3,4,5-trimethoxyphenyl)-1*H*-indole-5-sulfonamide,
1-methyl-*N*-(4-morpholinylacetyl)-*N*-(3,4,5-trimethoxyphenyl)-1*H*-indole-5-sulfonamide,
1-methyl-*N*-((4-methyl-1-piperazinyl)acetyl)-*N*-(3,4,5-trimethoxyphenyl)-1*H*-indole-5-sulfonamide, and
*N*-(4-(aminomethyl)benzoyl)-1-methyl-*N*-(3,4,5-trimethoxyphenyl)-1*H*-indole-5-sulfonamide.

10. A compound according to Claim 1, wherein L¹ is -OSO₂-.

11. A compound according to Claim 10 selected from the group consisting of 1*H*-indol-5-yl 3,4,5-trimethoxybenzenesulfonate and 1-methyl-1H-indol-5-yl 3,4,5,-trimethoxybenzenesulfonate.

12. A compound according to Claim 1 .for use as a therapeutic agent.

13. Use of a compound of Claim 1 for manufacturing a medicament for inhibiting polymerization of tubulin in a mammal in recognized need of such treatment.

14. Use of a compound of Claim 1 for manufacturing a medicament for treating cancer in a mammal in recognized need of such treatment.

15. A compound according to Claim 1 selected from the group consisting of
1-formyl-*N*-(3,4,5-trimethoxyphenyl)indoline-5-sulfonamide,
*N*-(3,4,5-trimethoxyphenyl)indoline-5-sulfonamide,
5-nitro-*N*-(3,4,5-trimethoxyphenyl)-1*H*-indole-3-sulfonamide,
1-methyl-*N*-(3,4,5-trimethoxyphenyl)indoline-5-sulfonamide,
1-methyl-5-nitro-*N*-(3,4,5-trimethoxyphenyl)-1*H*-indole-3-sulfonamide,
5-amino-1-methyl-*N*-(3,4,5-trimethoxyphenyl)-1*H* indole-3-sulfonamide,
5-amino-*N*-(3,4,5-trimethoxyphenyl)-1*H*-indole-3-sulfonamide,
*N*-(3,4,5-trimethoxyphenyl)-1*H*-indole-5-sulfonamide,
1-methyl-*N*-(3,4,5-trimethoxyphenyl)-1*H*-indole-5-sulfonamide,
*N*,1-dimethyl-N-(3,4,5-trimethoxyphenyl)-1*H*-indole-5-sulfonamide,
1*H*-indol-5-yl 3,4,5-trimethoxybenzenesulfonate,
1-ethyl-*N*-(3,4,5-trimethoxyphenyl)-1*H*-indole-5-sulfonamide,
*N*-(2-hydroxyethyl)-1-methyl-*N*-(3,4,5-trimethoxyphenyl)-1*H*-indole-5-sulfonamide,
*N*-(2-fluoroethyl)-1-methyl-*N*-(3,4,5-trimethoxyphenyl)-1*H*-indole-5-sulfonamide
*N*-ethyl-1-methyl-*N*-(3,4,5-trimethoxyphenyl)-1*H*-indole-5-sulfonamide,
*N*-((dimethylamino)acetyl)-1-methyl-*N*-(3,4,5-trimethoxyphenyl)-1*H*-indole-5-sulfonamide,
1-methyl-*N*-(((2*S*)-1-methylpyrrolidnyl)carbonyl)-*N*-(3,4,5-trimethoxyphenyl)-1*H*-indole-5-sulfonamide,
*N*-((2*S*)-2-(dimethylamino)-3-methylbutanoyl)-1-methyl-*N*-(3,4,5-trimethoxyphenyl)-1*H*-indole-5-sulfonamide,
*N*-((2*S*)-2-amino-3-methylbutanoyl)-1-methyl-*N*-(3,4,5-trimethoxyphenyl)-1*H*-indole-5-sulfonamide,
1-methyl-*N*-((2*S*)-2-methylamino)propanoyl)-*N*-(3,4,5-trimethoxyhenyl)-1*H*-indole-5-sulfonamide,
*N*-((2*S*)-2-amino-2-phenylethanoyl)-1-methyl *N*-(3,4,5-trimethoxyphenyl)-1*H*-indole-5-sulfonamide,
*N*-((2*S*)-2-amino-3-phenylpropanoyl)-1-methyl-*N*-(3,4,5-trimethoxyphenyl)-1*H*-indole-5-sulfonamide
1-methyl-*N*-((2*S*)-pyrrolidinylcarbonyl)-*N*-(3,4,5-trimethoxyphenyl)-1*H*-indole-5-sulfonamide,
*N*-((2*S*)-2,6-diaminohexanoyl)-1-methyl-*N*-(3,4,5-trimethoxyphenyl)-1*H*-indole-5-sulfonamide,
*N*-((2*S*)-2-amino-3-(1*H*-imidazol-5-yl)propanoyl)-1-methyl-*N*-(3,4,5-trimethoxyphenyl)-1*H*-indole-5-sulfonamide,
(2*S*)-2-amino-4-oxo-4-(3,4,5-trimethoxy((1-methyl-1*H*-indol-5-yl)sulfonyl)anilino)butanoic acid,
(3*S*)-3-amino-4-oxo-4-(3,4,5-trimethoxy((1-methyl-1*H*-indol-5-yl)sulfonyl)anilino)butanoic acid,
(2*S*)-2-amino-5-oxo-5-(3,4,5-trimethoxy((1-methyl-1*H*-indol-5-yl)sulfonyl)anilino)pentanoic acid,
(4*S*)-4-amino-5-oxo-5-(3,4,5-trimethoxy((1-methyl-1*H*-indol-5-yl)sulfonyl)anilino)pentanoic acid,
*N*-((bis(2-methoxyethyl)amino)acetyl)-1-methyl-*N*-(3,4,5-trimethoxyphenyl)-1*H*-indole-5-sulfonamide,
1-methyl-*N*-(4-morpholinylacetyl)-*N*-(3,4,5-trimethoxyphenyl)-1*H*-indole-5-sulfonamide,
1-methyl-*N*-((4-methyl-1-piperazinyl)acetyl)-*N*-(3,4,5-trimethoxyphenyl)-1*H*-indole-5-sulfonamide, and
*N*-(4-(aminomethyl)benzoyl)-1-methyl-*N*-(3,4,5-trimethoxyphenyl)-1*H*-indole-5-sulfonamide.

16. A compound according to Claim 15 which is
*N*-((dimethylamino)acetyl)-1-methyl-*N*-(3,4,5-trimethoxyphenyl)-1*H*-indole-5-sulfonamide.

17. A pharmaceutical composition comprising a compound according to Claim 1 and one or more non-toxic pharmaceutically acceptable carriers.

## Patentansprüche

1. Eine Verbindung mit der Formel (I) 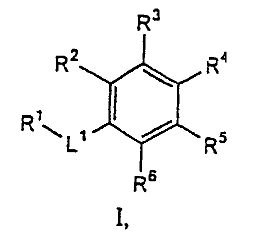 oder ein pharmazeutisch verträgliches Salz davon, worin L¹ gewählt ist aus der Gruppe bestehend aus
(1) -OS(O)₂-, und
(2) -SO₂NR⁷-, worin R⁷ gewählt ist aus der Gruppe bestehend aus
(a) Wasserstoff,
(b) Alkanoyl,
(c) Alkyl,
(d) Aryloyl, worin das Aryl Phenyl ist,
worin (b)-(d) wahlweise substituiert sein können mit ein oder zwei Substituenten unabhängig gewählt aus der Gruppe bestehend aus
(i) Hydroxyl,
(ii) Halo,
(iii) Carboxy,
(iv) Phenyl,
(v) Imidazolyl,
(vi) Heterocycloalkyl gewählt aus der Gruppe bestehend aus Morpholinyl und Piperazinyl,
(vii) -NR^{c}R^{d}, worin R^{c} und R^{d} unabhängig gewählt sind aus der Gruppe bestehend aus
(1') Wasserstoff,
(2') Alkyl,
und
(3') Alkoxyalkyl,
und
(viii) -(Alkylen)-NR^{c}R^{d},
worin für (v) und (vi) das Imidazol und das Heterocycloalkyl wahlweise substituiert sein kann mit einem Alkylsubstituenten,
und
(e) Pyrrolidinylcarbonyl, worin das Pyrrolidinylcarbonyl wahlweise substituiert sein kann mit einem Alkylsubstituenten,
worin (1)-(2) gezeigt sind mit ihren linken Enden gebunden an R¹ und ihren rechten Enden gebunden an den Phenylring;
R¹ ist Heteroaryl, worin das Heteroaryl gewählt ist aus der Gruppe bestehend aus Indolinyl und Indolyl, und worin das Heteroaryl wahlweise substituiert sein kann mit einem oder zwei Substituenten unabhängig gewählt aus der Gruppe bestehend aus
(a) Carboxaldehyd,
(b) Nitro,
(c) Alkyl, und
(d) -NH₂;
R² und R⁶ sind Wasserstoff;
und
R³, R⁴ und R⁵ sind Alkoxy.

2. Eine Verbindung gemäß Anspruch 1, worin L¹ -SO₂NR⁷- ist.

3. Eine Verbindung gemäß Anspruch 2, worin R³, R⁴ und R⁵ Methoxy sind.

4. Eine Verbindung gemäß Anspruch 3, worin R¹ wahlweise substituiertes 2,3-Dihydro-1*H*-indol-5-yl ist.

5. Eine Verbindung gemäß Anspruch 3, gewählt aus der Gruppe bestehend aus
1-Formyl-*N*-(3,4,5-trimethoxyphenyl)indolin-5-sulfonamid,
*N*-(3,4,5-Trimethoxyphenyl)indolin-5-sulfonamid, und
1-Methyl-*N*-(3,4,5-trimethoxyphenyl)indolin-5-sulfonamid.

6. Eine Verbindung gemäß Anspruch 3, worin R¹ wahlweise substituiertes 1*H*-Indol-3-yl ist.

7. Eine Verbindung gemäß Anspruch 6, gewählt aus der Gruppe bestehend aus
5-Nitro-*N*-(3,4,5-trimethoxyphenyl)-1*H*-indol-3-sulfonamid,
1-Methyl-5-nitro-*N*-(3,4,5-trimethoxyphenyl)-1*H*-indol-3-sulfonamid,
5-Amino-1-methyl-*N*-(3,4,5-trimethoxyphenyl)-1*H*-indol-3-sulfonamid, und
5-Amino-*N*-(3,4,5-trimethoxyphenyl)-1*H*-indol-3-sulfonamid.

8. Eine Verbindung gemäß Anspruch 3, worin R¹ wahlweise substituiertes 1*H*-Indol-5-yl ist.

9. Eine Verbindung gemäß Anspruch 8, gewählt aus der Gruppe bestehend aus
*N*-(3,4,5-Trimethoxyphenyl)-1*H*-indol-5-sulfonamid,
1-Methyl-*N*-(3,4,5-trimethoxyphenyl-1*H*-indol-5-sulfonamid,
*N*,1-Dimethyl-*N*-(3,4,5-trimethoxyphenyl)-1*H*-indol-5-sulfonamid,
1-Ethyl-*N*-(3,4,5-trimethoxyphenyl)-1*H*-indol-5-sulfonamid,
*N*-(2-Hydroxyethyl)-1-methyl-*N*-(3,4,5-trimethoxyphenyl)-1*H*indol-5-sulfonamid,
*N*-(2-Fluorethyl)-1-methyl-*N*-(3,4,5-trimethoxyphenyl)-1*H*-indol-5-sulfonamid,
*N*-Ethyl-1-methyl-*N*-(3,4,5-trimethoxyphenyl)-1*H*-indol-5-sulfonamid,
*N*-((Dimethylamino)acetyl)-1-methyl-*N*-(3,4,5-trimethoxyphenyl)-1*H*-indol-5-sulfonamid,
1-Methyl-*N*-(((2*S*)-1-methylpyrrolidinyl)carbonyl)-*N*-(3,4,5-trimethoxyphenyl)-1*H*-indol-5-sulfonamid,
*N*-((2*S*)-2-(Dimethylamino)-3-methylbutanoyl)-1-methyl-*N*-(3,4,5-trimethoxyphenyl)-1*H*-indol-5-sulfonamid,
*N*-((2*S*)-2-Amino-3-methylbutanoyl)-1-methyl-*N*-(3,4,5-trimethoxyphenyl)-1*H*-indol-5-sulfonamid,
1-Methyl-*N*-((2S)-2-methylamino)propanoyl)-*N*-(3,4,5-trimethoxyphenyl)-1*H*-indol-5-sulfonamid,
*N*-((2*S*)-2-Amino-2-phenylethanoyl)-1-methyl-*N*-(3,4,5-trimethoxyphenyl)-1*H*-indol-5-sulfonamid,
*N*-((2*S*)-2-Amino-3-phenylpropanoyl)-1-methyl-*N*-(3,4,5-trimethoxyphenyl)-1*H*-indol-5-sulfonamid,
1-Methyl-*N*-((2*S*)-2-pyrrolidinylcarbonyl)-*N*-(3,4,5-trimethoxyphenyl)-1*H*-indol-5-sulfonamid,
*N*-((2*S*)-2,6-Diaminohexanoyl)-1-methyl-*N*-(3,4,5-trimethoxyphenyl)-1*H*-indol-5-sulfonamid,
*N*-((2*S*)-2-Amino-3-(1H-imidazol-5-yl)propanoyl)-1-methyl-*N*-(3,4,5-trimethoxyphenyl)-1*H*-indol-5-sulfonamid,
(2*S*)-2-Amino-4-oxo-4-(3,4,5-trimethoxy((1-methyl-1*H*-indol-5-yl)sulfonyl)anilin)buttersäure,
(3*S*)-3-Amino-4-oxo-4-(3,4,5-trimethoxy((1-methyl)-1*H*-indol-5-yl)sulfonyl)anilin)buttersäure,
(2*S*)-2-Amino-5-oxo-5-(3,4,5-trimethoxy((1-methyl-1*H*-indol-5-yl)sulfonyl)anilin)pentansäure,
(4*S*)-4-Amino-5-oxo-5-(3,4,5-trimethoxy((1-methyl-1*H*-indol-5-yl)sulfonyl)anilin)pentansäure,
*N*-((bis(2-Methoxyethyl)amino)acetyl)-1-methyl-*N*-(3,4,5-trimethoxyphenyl)-1*H*-indol-5-sulfonamid,
1-Methyl-*N*-(4-morpholinylacetyl)-*N*-(3,4,5-trimethoxyphenyl)-1*H*indol-5-sulfonamid,
1-Methyl-*N*-((4-methyl-1-piperazinyl)acetyl)-*N*-(3,4,5-trimethoxyphenyl)-1*H*-indol-5-sulfonamid, und
*N*-(4-(Aminomethyl)benzoyl)-1-methyl-*N*-(3,4,5-trimethoxyphenyl)-1*H*-indol-5-sulfonamid.

10. Eine Verbindung gemäß Anspruch 1, worin L¹ -OSO₂- ist.

11. Eine Verbindung gemäß Anspruch 10, gewählt aus der Gruppe bestehend aus 1*H*-Indol-5-yl 3,4,5-Trimethoxybenzensulfonat und 1-Methyl-1H-indol-5-yl 3,4,5-Trimethoxybenzensulfonat.

12. Eine Verbindung gemäß Anspruch 1, zur Verwendung als ein therapeutischer Wirkstoff.

13. Verwendung einer Verbindung von Anspruch 1, zur Herstellung eines Medikaments zur Hemmung der Polymerisation von Tubulin in einem Säugetier, von dem erkannt wird, dass es einer solchen Behandlung bedarf.

14. Verwendung einer Verbindung von Anspruch 1, zur Herstellung eines Medikaments zur Behandlung von Krebs in einem Säugetier, von dem erkannt wird, dass es einer solchen Behandlung bedarf.

15. Eine Verbindung gemäß Anspruch 1, gewählt aus der Gruppe bestehend aus
1-Formyl-*N*-(3,4,5-trimethoxyphenyl)indolin-5-sulfonamid,
*N*-(3,4,5-Trimethoxyphenyl)indolin-5-sulfonamid,
5-Nitro-*N*-(3,4,5-trimethoxyphenyl)-1*H*-indol-3-sulfonamid,
1-Methyl-*N*-(3,4,5-trimethoxyphenyl)indolin-5-sulfonamid,
1-Methyl-5-nitro-*N*-(3,4,5-trimethoxyphenyl)-1*H*-indol-3-sulfonamid,
5-Amino-1-methyl-*N*-(3,4,5-trimethoxyphenyl)-1*H*-indol-3-sulfonamid,
5-Amino-*N*-(3,4,5-trimethoxyphenyl)-1*H*-indol-3-sulfonamid,
*N*-(3,4,5-Trimethoxyphenyl)-1*H*-indol-5-sulfonamid,
1-Methyl-*N*-(3,4,5-trimethoxyphenyl)-1*H*-indol-5-sulfonamid,
*N*,1-Dimethyl-*N*-(3,4,5-trimethoxyphenyl)-1*H*-indol-5-sulfonamid,
1*H*-Indol-5-yl 3,4,5-Trimethoxybenzensulfonat,
1-Ethyl-*N*-(3,4,5-trimethoxyphenyl)-1*H*-indol-3-sulfonamid,
*N*-(2-Hydroxyethyl)-1-methyl-*N*-(3,4,5-trimethoxyphenyl)-1*H*indol-5-sulfonamid,
*N*-(2-Fluorethyl)-1-methyl-*N*-(3,4,5-trimethoxyphenyl)-1*H*-indol-5-sulfonamid,
*N*-Ethyl-1-methyl-*N*-(3,4,5-trimethoxyphenyl)-1*H*-indol-5-sulfonamid,
*N*-((Dimethylamino)acetyl)-1-methyl-*N*-(3,4,5-trimethoxyphenyl)-1*H*-indol-5-sulfonamid,
1-Methyl-*N*-(((2*S*)-1-methylpyrrolidinyl)carbonyl)-*N*-(3,4,5-trimethoxyphenyl)-1*H*-indol-5-sulfonamid,
*N*-((2*S*)-2-(Dimethylamino)-3-methylbutanoyl)-1-methyl-*N*-(3,4,5-trimethoxyphenyl)-1*H*-indol-5-sulfonamid,
*N*-((2*S*)-2-Amino-3-methylbutanoyl)-1-methyl-*N*-(3,4,5-trimethoxyphenyl)-1*H*-indol-5-sulfonamid,
1-Methyl-*N*-((2*S*)-2-methylamino)propanoyl)-*N*-(3,4,5-trimethoxyphenyl)-1*H*-indol-5-sulfonamid,
*N*-((2*S*)-2-Amino-2-phenylethanoyl)-1-methyl-*N*-(3,4,5-trimethoxyphenyl)-1*H*-indol-5-sulfonamid,
*N*-((2*S*)-2-Amino-3-phenylpropanoyl)-1-methyl-*N*-(3,4,5-trimethoxyphenyl)-1*H*-indol-5-sulfonamid,
1-Methyl-*N*-((2*S*)-pyrrolidinylcarbonyl)-*N*-(3,4,5-trimethoxyphenyl)-1*H*-indol-5-sulfonamid,
*N*-((2*S*)-2,6-Diaminohexanoyl)-1-methyl-*N*-(3,4,5-trimethoxyphenyl)-1*H*-indol-5-sulfonamid,
*N*-((2*S*)-2-Amino-3-(1*H*-imidazol-5-yl)propanoyl)-1-methyl-*N*-(3,4,5-trimethoxyphenyl)-1*H*-indol-5-sulfonamid,
(2*S*)-2-Amino-4-oxo-4-(3,4,5-trimethoxy((1-methyl)-1*H*-indol-5-yl)sulfonyl)anilin)buttersäure,
(3*S*)-3-Amino-4-oxo-4-(3,4,5-trimethoxy((1-methyl-1*H*-indol-5-yl)sulfonyl)anilino)buttersäure,
(2*S*)-2-Amino-5-oxo-5-(3,4,5-trimethoxy((1-methyl-1*H*-indol-5-yl)sulfonyl)anilino)pentansäure,
(4*S*)-4-Amino-5-oxo-5-(3,4,5-trimethoxy((1-methyl-1*H*-indol-5-yl)sulfonyl)anilino)pentansäure,
*N*-((bis(2-Methoxyethyl)amino)acetyl)-1-methyl-*N*-(3,4,5-trimethoxyphenyl)-1*H*-indol-5-sulfonamid,
1-Methyl-*N*-(4-morpholinylacetyl)-*N*-(3,4,5-trimethoxyphenyl)-1*H*indol-5-sulfonamid,
1-Methyl-*N*-((4-methyl-1-piperazinyl)acetyl)-*N*-(3,4,5-trimethoxyphenyl)-1*H*-indol-5-sulfonamid, und
*N*-(4-(Aminomethyl)benzoyl)-1-methyl-*N*-(3,4,5-trimethoxyphenyl)-1*H*-indol-5-sulfonamid.

16. Eine Verbindung gemäß Anspruch 15, die *N*-((Dimethylamino)acetyl)-1-methyl-*N*-(3,4,5-trimethoxyphenyl)-1*H*-indol-5-sulfonamid ist.

17. Eine pharmazeutische Zusammensetzung, die eine Verbindung gemäß Anspruch 1 und einen oder mehrere nicht toxische pharmazeutisch verträgliche Träger umfaßt.

## Revendications

1. Composé de formule (I) 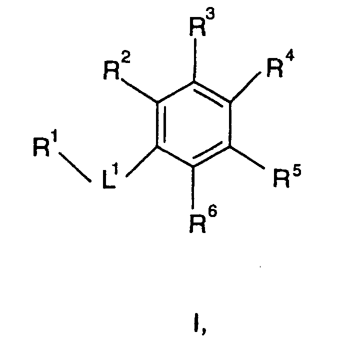 ou un sel pharmaceutiquement acceptable de celui-ci, où L¹ est choisi dans le groupe consistant en :
(1) -OS(O)₂-, et
(2) -SO₂NR⁷-, où R⁷ est choisi dans le groupe consistant en :
(a) l'hydrogène,
(b) un groupe alcanoyle,
(c) un groupe alkyle,
(d) un groupe aryloyle, dans lequel le groupe aryle est phényle,
où (b) à (d) peuvent être facultativement substitués par un ou deux substituants choisis indépendamment du groupe consistant en :
(i) un groupe hydroxyle,
(ii) un groupe halogéno,
(iii) un groupe carboxy,
(iv) un groupe phényle,
(v) un groupe imidazolyle,
(vi) un groupe hétérocycloalkyle choisi dans le groupe consistant en un groupe morpholinyle et pipérazinyle,
(vii) -NR^{c}R^{d}, où R^{c} et R^{d} sont choisis indépendamment dans le groupe consistant en :
(1') l'hydrogène,
(2') un groupe alkyle,
et
(3') un groupe alcoxyalkyle,
et
(viii) -(alkylène)-NR^{c}R^{d},
où, pour (v) et (vi), les groupes imidazolyle et hétérocycloalkyle peuvent être facultativement substitués par un substituant alkyle, et
(e) un groupe pyrrolidinylcarbonyle, où le groupe pyrrolidinylcarbonyle peut être facultativement substitué par un substituant alkyle,
où (1) et (2) sont représentés avec leurs extrémités à gauche fixées à R¹ et leurs extrémités à droite, fixées au cycle phénylique ;
R¹ est un groupe hétéroaryle, dans lequel le groupe hétéroaryle est choisi dans le groupe consistant en les groupes indolinyle et indolyle, et dans lequel le groupe hétéroaryle peut être éventuellement substitué par un ou deux substituants indépendamment choisis dans le groupe consistant en :
(a) un carboxaldéhyde,
(b) un groupe nitro,
(c) un groupe alkyle, et
(d) -NH₂ ;
R² et R⁶ sont l'hydrogène ;
et
R³, R⁴ et R⁵ sont un groupe alcoxy.

2. Composé selon la revendication 1, dans lequel L¹ est -SO₂NR⁷-.

3. Composé selon la revendication 2, dans lequel R³, R⁴ et R⁵ sont un groupe méthoxy.

4. Composé selon la revendication 3, dans lequel R¹ est un groupe 2,3-dihydro-1*H*-indol-5-yle facultativement substitué.

5. Composé selon la revendication 3, choisi dans le groupe consistant en du 1-formyl-*N*-(3,4, 5-triméthoxyphényl)indoline-5-sulfonamide, du *N*-(3,4,5-triméthoxyphényl)indoline-5-sulfonamide, et du 1-méthyl-*N*-(3,4,5-triméthoxyphényl)indoline-5-sulfonamide.

6. Composé selon la revendication 3, dans lequel R¹ est un groupe 1*H*-indol-3-yle facultativement substitué.

7. Composé selon la revendication 6, choisi dans le groupe consistant en du 5-nitro-*N*-(3,4,5-triméthoxyphényl)-1*H*-indole-3-sulfonamide, du 1-méthyl-5-nitro-*N*-(3,4,5-triméthoxyphényl-1*H*-indole-3-sulfonamide, du 5-amino-1-méthyl-*N*-(3,4,5-triméthoxyphényl)-1*H*-indole-3-sulfonamide, et du 5-amino-*N*-(3,4,5-triméthoxyphényl)-1*H*-indole-3-sulfonamide.

8. Composé selon la revendication 3, dans lequel R¹ est un groupe 1*H*-indol-5-yle facultativement substitué.

9. Composé selon la revendication 8, choisi dans le groupe consistant en du *N*-(3,4,5-triméthoxyphényl)-1*H*-indole-5-sulfonamide,
du 1-méthyl-*N*-(3,4,5-triméthoxyphényl)-1*H*-indole-5-sulfonamide,
du *N*,1-diméthyl-*N*-(3,4,5-triméthoxyphényl)-1*H*-indole-5-sulfonamide,
du 1-éthyl-*N*-(3,4,5-triméthoxyphényl)-1*H*-indole-5-sulfonamide,
du *N*-(2-hydroxyéthyl)-1-méthyl-*N*-(3,4,5-triméthoxyphényl)-1*H*-indole-5-sulfonamide,
du *N*-(2-fluoroéthyl)-1-méthyl-*N*-(3,4,5-triméthoxyphényl)-1*H*-indole-5-sulfonamide,
du *N*-éthyl-1-méthyl-*N*-(3,4,5-triméthoxyphényl)-1*H*-indole-5-sulfonamide,
du *N*-((diméthylamino)acétyl)-1-méthyl-*N*-(3,4,5-triméthoxyphényl)-1*H*-indole-5-sulfonamide,
du 1-méthyl-*N*-(((2S)-1-méthylpyrrolidinyl)carbonyl)-*N*-(3,4,5-triméthoxyphényl)-1*H*-indole-5-sulfonamide,
du *N*-((2S)-2-(diméthylamino)-3-méthylbutanoyl)-1-méthyl-*N*-(3,4,5-triméthoxyphényl)-1*H*-indole-5-sulfonamide,
du *N*-((2S)-2-amino-3-méthylbutanoyl)-1-méthyl-*N*-(3,4,5-triméthoxyphényl)-1*H*-indole-5-sulfonamide,
du 1-méthyl-*N*-((2S)-2-méthylamino)propanoyl)-*N*-(3,4,5-triméthoxyphényl)-1*H*-indole-5-sulfonamide,
du *N*-((2S)-2-amino-2-phényléthanoyl)-1-méthyl-*N*-(3,4,5-triméthoxyphényl)-1*H*-indole-5-sulfonamide,
du *N*-((2S)-2-amino-3-phénylpropanoyl)-1-méthyl-*N*-(3,4,5-triméthoxyphényl)-1*H*-indole-5-sulfonamide,
du 1-méthyl-*N*-((2S)-pyrrolidinylcarbonyl)-*N*-(3,4,5-triméthoxyphényl)-1*H*-indole-5-sulfonamide,
du *N*-((2*S*)-2,6-diaminohexanoyl)-2-méthyl-*N*-(3,4,5-triméthoxyphényl)-1*H*-indole-5-sulfonamide,
du *N*-((2S)-2-amino-3-(1*H*-imidazol-5-yl)propanoyl)-1-méthyl-*N*-(3,4,5-triméthoxyphényl)-1*H*-indole-5-sulfonamide,
de l'acide (2S)-2-amino-4-oxo-4-(3,4,5-triméthoxy((1-méthyl-1*H*-indol-5-yl)sulfonyl)anilino)butanoique,
de l'acide (3S)-3-amino-4-oxo-4-(3,4,5-triméthoxy((1-méthyl-1*H*-indol-5-yl)sulfonyl)anilino)butanoïque,
de l'acide (2S)-2-amino-5-oxo-5-(3,5,4-triméthoxy((1-méthyl-1*H*-indol-5-yl)sulfonyl)anilino)pentanoïque,
de l'acide (4S)-4-amino-5-oxo-5-(3,4,5-triméthoxy((1-méthyl-1*H*-indol-5-yl)sulfonyl)anilino)pentanoïque,
du *N*-((bis(2-méthoxyéthyl)amino)acétyl)-1-méthyl-*N*-(3,4,5-triméthoxyphényl)-1*H*-indole-5-sulfonamide,
du 1-méthyl-*N*-(4-morpholinylacétyl)-*N*-(3,4,5-triméthoxyphényl)-1H-indole-5-sulfonamide,
du 1-méthyl-*N*-((4-méthyl-1-pipérazinyl)acétyl)-*N*-(3,4,5-triméthoxyphényl-1*H*-indole-5-sulfonamide, et
du *N*-(4-(aminométhyl)benzoyl)-1-méthyl-*N*-(3,4,5-triméthoxyphényl)-1*H*-indole-5-sulfonamide.

10. Composé selon la revendication 1, dans lequel L¹ est -OSO₂-.

11. Composé selon la revendication 10, choisi dans le groupe consistant en du 1H-indol-5-yl 3,4,5-triméthoxybenzènesulfonate et du 1-méthyl-1H-indol-5-yl 3,4,5-triméthoxybenzènesulfonate.

12. Composé selon la revendication 1, destiné être utilisé en tant qu'agent thérapeutique.

13. Utilisation d'un composé selon la revendication 1, destiné à la fabrication d'un médicament pour inhiber la polymérisation de la tubuline chez un mammifère lors d'un besoin avéré de ce traitement.

14. Utilisation d'un composé selon la revendication 1, pour fabriquer un médicament destiné au traitement du cancer chez un mammifère lors d'un besoin avéré de ce traitement.

15. Composé selon la revendication 1, choisi dans le groupe consistant en :
du 1-formyl-*N*-(3,4,5-triméthoxyphényl)indoline-5-sulfonamide,
du *N*-(3,4,5-triméthoxyphényl)indoline-5-sulfonamide,
du 5-nitro-*N*-(3,4,5-triméthoxyphényl)-1H-indole-3-sulfonamide,
du 1-méthyl-*N*-(3,4,5-triméthoxyphényl)indoline-5-sulfonamide,
du 1-méthyl-5-nitro-*N*-(3,4,5-triméthoxyphényl)-1*H*-indole-3-sulfonamide,
du 5-amino-1-méthyl-*N*-(3,4,5-triméthoxyphényl)-1*H*-indole-3-sulfonamide,
du 5-amino-*N*-(3,4,5-triméthoxyphényl)-1*H*-indole-5-sulfonamide,
du *N*-(3,4,5-triméthoxyphényl)-1*H*-indole-5-sulfonamide,
du 1-méthyl-*N*-(3,4,5-triméthoxyphényl)-1*H*-indole-5-sulfonamide,
du *N*,1-diméthyl-*N*-(3,4,5-triméthoxyphényl)-1*H*-indole-5-sulfonamide,
du 1*H*-indol-5-yl-3,4,5-triméthoxybenzènesulfonate,
du 1-éthyl-(3,4,5-triméthoxyphényl)-1*H*-indole-5-sulfonamide,
du *N*-(2-hydroxyéthyl)-1-méthyl-*N*-(3,4,5-triméthoxyphényl)-1*H*-indole-5-sulfonamide,
du *N*-(2-fluoroéthyl)-1-méthyl-*N*-(3,4,5-triméthoxyphényl)-1*H*-indole-5-sulfonamide,
du *N*-((diméthylamino)acétyl)-1-méthyl-*N*-(3,4,5-triméthoxyphényl)-1*H*-indole-5-sulfonamide,
du *N*-((diméthylamino)acétyl)-1-méthyl-*N*-(3,4,5-triméthoxyphényl)-1*H*-indole-5-sulfonamide,
du 1-méthyl-*N*-(((2*S*)-1-méthylpyrrolidinyl)carbonyl)-*N*-(3,4,5-triméthoxyphényl)-1*H*-indole-5-sulfonamide,
du *N*-((2S)-2-(diméthylamino)-3-méthylbutanoyl)-1-méthyl-*N*-(3,4,5-triméthoxyphényl)-1*H*-indole-5-sulfonamide,
du *N*-((2S)-2-amino-3-méthylbutanoyl)-1-méthyl-*N*-(3,4,5-triméthoxyphényl)-1*H*-indole-5-sulfonamide,
du 1-méthyl-*N*-((2S)-2-méthylamino)propanoyl)-*N*-(3,4,5-triméthoxyphényl)-1*H*-indole-5-sulfonamide,
du *N*-((2S)-2-amino-2-phényléthanoyl)-1-méthyl-*N*-(3,4,5-triméthoxyphényl)-1*H*-indole-5-sulfonamide,
du *N*-((2S)-2-amino-2-phénylpropanoyl)-1-méthyl-*N*-(3,4,5-triméthoxyphényl)-1*H*-indole-5-sulfonamide,
du 1-méthyl-*N*-((2S)-1-méthylpyrrolidinyl)carbonyl-*N*-(3,4,5-triméthoxyphényl)-1*H*-indole-5-sulfonamide,
du *N*-((2S)-2,6-diaminohexanoyl)-1-méthyl-*N*-(3,4,5-triméthoxyphényl)-1*H*-indole-5-sulfonamide,
du *N*-((2S)-2-amino-3-(1*H*-imidazol-5-yl)propanoyl)-1-méthyl-*N*-(3,4,5-triméthoxyphényl)-1*H*-indole-5-sulfonamide,
de l'acide (2S)-2-amino-4-oxo-4-(3,4,5-triméthoxy((1-méthyl-1*H*-indol-5-yl)sulfonyl)anilino)butanoïque,
de l'acide (2S)-3-amino-4-oxo-4-(3,4,5-triméthoxy((1-méthyl-1*H*-indol-5-yl)sulfonyl)anilino)butanoïque,
de l'acide (2S)-2-amino-5-oxo-5-(3,4,5-triméthoxy((1-méthyl-1*H*-indol-5-yl)sulfonyl)anilino)pentanoique,
de l'acide (4S)-4-amino-5-oxo-5-(3,4,5-triméthoxy((1-méthyl-1*H*-indol-5-yl)sulfonyl)anilino)pentanoïque,
du *N*-((bis(2-méthoxyéthyl)amino)acétyl)-1-méthyl-*N*-3,4,5-triméthoxyphényl)-1*H*-indole-5-sulfonamide,
du 1-méthyl-*N*-(4-morpholinylacétyl)-*N*-(3,4,5-triméthoxyphényl)-1*H*-indole-5-sulfonamide,
du 1-méthyl-*N*-((4-méthyl-1-pipérazinyl)acétyl)-*N*-(3,4,5-triméthoxyphényl)-1*H*-indole-5-sulfonamide, et
du *N*-(4-(aminométhyl)benzoyl)-1-méthyl-*N*-(3,4,5-triméthoxyphényl)-1*H*-indole-5-sulfonamide.

16. Composé selon la revendication 15, qui est le N-((diméthylamino)acétyl)-1-méthyl-*N*-(3,4,5-triméthoxyphényl)-1H-indole-5-sulfonamide.

17. Composition pharmaceutique comprenant un composé selon la revendication 1, et un ou plusieurs véhicules non toxiques pharmaceutiquement acceptables.
